(19) **Europäisches Patentamt / European Patent Office / Office européen des brevets**

(11) **EP 3 921 776 B1**

(12) **EUROPEAN PATENT SPECIFICATION**

(45) Date of publication and mention of the grant of the patent:
**07.08.2024 Bulletin 2024/32**

(21) Application number: **20752736.7**

(22) Date of filing: **07.02.2020**

(51) International Patent Classification (IPC):
**G06T 7/00** *(2017.01)* **G06N 3/084** *(2023.01)*
**G06T 19/20** *(2011.01)* **G06V 10/44** *(2022.01)*
**G06V 10/82** *(2022.01)* **G06V 20/64** *(2022.01)*

(52) Cooperative Patent Classification (CPC):
**G16H 50/20; G06F 18/2431; G06N 3/045;
G06N 3/084; G06T 7/0012; G06T 19/20;
G06V 10/454; G06V 10/82; G06V 20/647;
G16H 30/40;** G06T 2207/10101; G06T 2207/20016;
G06T 2207/20081; G06T 2207/20084;
G06T 2207/30041; (Cont.)

(86) International application number:
**PCT/SG2020/050057**

(87) International publication number:
**WO 2020/162834 (13.08.2020 Gazette 2020/33)**

(54) **METHOD AND SYSTEM FOR CLASSIFICATION AND VISUALISATION OF 3D IMAGES**

VERFAHREN UND SYSTEM ZUR KLASSIFIZIERUNG UND VISUALISIERUNG VON 3D-BILDERN

PROCÉDÉ ET SYSTÈME DE CLASSIFICATION ET DE VISUALISATION D'IMAGES 3D

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2019 SG 10201901083Y**

(43) Date of publication of application:
**15.12.2021 Bulletin 2021/50**

(73) Proprietors:
• **Singapore Health Services Pte. Ltd.**
**Singapore 168753 (SG)**
• **Agency for Science, Technology and Research**
**Singapore 138632 (SG)**

(72) Inventors:
• **TING, Daniel Shu Wei**
**Singapore 168753 (SG)**
• **TAN, Gavin Siew Wei**
**Singapore 168753 (SG)**
• **WONG, Tien Yin**
**Singapore 168753 (SG)**
• **CHENG, Ching-Yu**
**Singapore 168753 (SG)**

• **CHEUNG, Chui Ming Gemmy**
**Singapore 168753 (SG)**
• **LIU, Yong**
**Singapore 138632 (SG)**
• **LI, Shaohua**
**Singapore 138632 (SG)**
• **GOH, Rick Siow Mong**
**Singapore 138632 (SG)**

(74) Representative: **Gill Jennings & Every LLP
The Broadgate Tower
20 Primrose Street
London EC2A 2ES (GB)**

(56) References cited:
**CN-A- 108 492 297      US-A1- 2018 018 553
US-A1- 2018 061 058      US-A1- 2018 082 443
US-A1- 2018 365 532**

**(Cont. next page)**

- **SELVARAJU RAMPRASAATH R ET AL: "Grad-CAM: Visual Explanations from Deep Networks via Gradient-Based Localization", 2017 IEEE INTERNATIONAL CONFERENCE ON COMPUTER VISION (ICCV), IEEE, 21 March 2017 (2017-03-21), pages 618 - 626, XP033282917, DOI: 10.1109/ICCV.2017.74**
- **KOOMSUBHA THANABHAT ET AL: "A character-level convolutional neural network with dynamic input length for Thai text categorization", 2017 9TH INTERNATIONAL CONFERENCE ON KNOWLEDGE AND SMART TECHNOLOGY (KST), IEEE, 1 February 2017 (2017-02-01), pages 101 - 105, XP033080591, DOI: 10.1109/KST.2017.7886102**
- **MAX JADERBERG ET AL: "Spatial Transformer Networks", 5 June 2015 (2015-06-05) - 13 October 2022 (2022-10-13), pages 1 - 14, XP080795056, Retrieved from the Internet <URL:https://arxiv.org/abs/1506.02025v1>**
- **ALEXANDER KATZMANN ET AL: "Predicting Lesion Growth and Patient Survival in Colorectal Cancer Patients using Deep Neural Networks", 1ST CONFERENCE ON MEDICAL IMAGING WITH DEEP LEARNING (MIDL 2018), 11 April 2018 (2018-04-11), Amsterdam, The Netherlands, pages 1 - 12, XP055664781, Retrieved from the Internet <URL:https://openreview.net/pdf?id=B1e_uuoif> [retrieved on 20200204]**

(52) Cooperative Patent Classification (CPC): (Cont.)
G06T 2210/41; G06T 2219/2012; G06V 2201/03

**Description**

**TECHNICAL FIELD**

The present disclosure relates to methods and systems for training image classifiers, methods and systems for classification using trained image classifiers, and methods and systems for visualising regions of interest in classified 3D images. It has particular, but not exclusive, applicability to the computer-aided diagnosis (CAD) of diseases in 3D medical images. Embodiments relate to detecting diseases, and localising and visualising abnormal regions in 3D medical images using deep learning methods.

**BACKGROUND**

3D medical imaging is a technique that creates visual representations of the interior body for medical analysis, and enables medical specialists to diagnose ailments precisely. The global 3D medical imaging services market was valued at $149,492 million in 2016, and is expected to reach $236,809 million by 2023 at a compound annual growth rate of 6.7% during the forecast period. The adoption of 3D medical imaging has increased over the years owing to a rapid increase in the use of point-of-care imaging, coupled with an increase in the elderly population and prevalence of chronic diseases globally.

One barrier to adoption of 3D imaging for medical applications is the lack of radiologists who are skilled in the technique. Accordingly, computer-aided diagnosis (CAD) has emerged as one way to address this issue. CAD, as an image classification task, has traditionally been implemented by extracting hand-crafted low-level image features, and feeding the features into a general-purpose classifier. However, the accuracy of such approaches has been found to be far below that of radiologists.

In recent years, alternative approaches have used deep learning methods on medical images, and have achieved excellent performance that is on a par with or even better than average radiologists. The superior performance of deep learning has mainly been attributed to the use of high-level feature extractors trained on large data sets that effectively capture discriminative patterns regardless of small deformations, position translations, scale, lighting and colour variations.

One of the biggest challenges of deep learning-based CAD systems is that a deep learning model, such as a Convolutional Neural Network (CNN), has millions of parameters, which usually can only be well optimised on a large set of fully-annotated training data. For example, in work by Valente et al. (Computer Methods and Programs in Biomedicine, Volume 124, 2016, Pages 91-107, ISSN 0169-2607) and Kuruvilla et al. (Computer Methods and Programs in Biomedicine. 113, 1 (January 2014), 202-209), deep learning models were trained on more than 100,000 2D images. Training data sets of this size are typically unavailable. In the most common scenarios, only thousands or tens of thousands of training images may be available.

One known practice for alleviating the required amount of training data is to fine-tune the parameters of a deep learning model that is pre-trained on a large set of natural images. However, when considering 3D image data, such as Retinal Optical Coherence Tomography (OCT) images, even after applying model fine-tuning, the data scarcity problem is still severe, for at least two reasons. Firstly, 3D imaging is usually more expensive than 2D imaging, and the available 3D images are often only in the hundreds or thousands. Secondly, each 3D image consists of many 2D images (slices), and typically only an overall label (healthy or diseased) for a whole 3D image is applied by radiologists, without any discrimination between normal and abnormal 2D slices of the 3D image.

When each of the 3D images is only provided with an overall label, this task is referred to as "Multiple Instance Learning", which is a subclass of "weakly-supervised" or "weakly-annotated" learning tasks. A straightforward approach to classifying 3D images is to use a 3D-CNN that captures 3D patterns. However a 3D-CNN has many more parameters to be optimized than the usual 2D-CNN. Accordingly, training a 3D-CNN requires a huge amount of 3D training images, so is not practical in CAD applications.

A further challenge of deep learning-based CAD systems is that the images used for training and diagnosis are often acquired from different device models or by different imaging protocols. For convenience of explanation, the two situations are collectively referred to as "from different devices". Two typical situations are that the training and diagnosed images (referred to as "test images" in the following) could be from different devices, and that the training images are a mixture acquired from multiple devices. Naturally, images from different devices may have drastically different device characteristics, including intensity profiles and resolutions. If the test images and the training images are collected from different devices, the CAD system may experience a severe performance drop when put in use. If the training images are a mixture of images from different devices, the model may have difficulty in adapting to the different device characteristics, and capturing the common patterns in images from all the devices. Hence a CAD system that automatically adapts to different devices would be advantageous.

A yet further challenge of deep learning on medical images, as in other applications of deep learning, is interpretability. Medical doctors may be reluctant to accept the diagnosis of CADs before understanding the basis of the diagnosis. It would therefore be advantageous to localise and visualise the detection results, i.e., to highlight the automatically detected pathological areas. There have been a few previous attempts to visualise deep learning classification results, but they either require revising and retraining the model, or do not pinpoint precise areas to be highlighted.

Examples of image process technologies include that disclosed in US 2018/082443, which relates to anomaly detection in volumetric (3D) images using a 2D CNN to encode slices within a volumetric image, such as a CT scan. There are also exploratory methodologies disclosed in Selvaraju Ramprasaath R et al's "Grad-CAM: Visual Explanations from Deep Networks via Gradient-Based Localization", Koomsubha Thanabhat et al's "A character-level convolutional neural network with dynamic input length for Thai text categorisation" and Max Jaderberg et al's "Spatial Transformer Networks" that can be used in such technologies.

It is desirable therefore to provide a method and system that overcomes or alleviates one or more of the above difficulties, or which at least provides a useful alternative.

## SUMMARY

The present invention is defined by the features disclosed in the independent claims. Additional embodiments are defined in the dependent claims. The present invention provides a computer-aided diagnosis (CAD) system for classification and visualisation of a 3D medical image, as disclosed in claim 1.

The present invention also provides a computer-aided diagnosis (CAD) method, as disclosed in claim 6.

The present invention further provides a non-volatile computer-readable storage medium having instructions stored thereon for causing at least one processor to perform the method of claim 6.

## BRIEF DESCRIPTION OF THE DRAWINGS

Some embodiments of a method and system for classification of images, and training of classifiers for that purpose, in accordance with present teachings will now be described, by way of non-limiting example only, with reference to the accompanying drawings in which:

Figure 1 shows an example block architecture of a neural network-based classification and visualisation system;
Figure 2 shows, in schematic form, further details of the architecture of a classification component of the system of Figure 1;
Figure 3 illustrates a pooling process implemented in methods according to certain embodiments;
Figure 4 illustrates a feature reweighting component according to certain embodiments;
Figure 5 schematically illustrates operative features of a back-propagation based visualisation component according to certain embodiments;
Figure 6 is an example architecture of a computer system for implementing certain embodiments;
Figure 7 is a flow diagram of an example method for training a classifier;
Figure 8 shows a Receiver Operating Characteristic (ROC) curve obtained for an example classifier trained only on 3D images;
Figure 9 shows a Receiver Operating Characteristic (ROC) curve obtained for an example classifier trained on a mixture of 2D and 3D images; and
Figure 10 shows an example of a visualisation using the visualisation component of Figure 5.

## DETAILED DESCRIPTION

Embodiments of the present disclosure relate to the automatic diagnosis of 3D medical images. Various embodiments may address one or more of the three challenges described above, and may enable 1) training a deep learning model on a small number of training images with only weak annotations available; 2) deploying the deep learning model on test images that have different characteristics than the training images due to different acquisition devices, which may otherwise cause severe degradation of model performance if treated without distinction; 3) visualising the detected pathological areas in the medical image, to help doctors verify the model output and establish reliable diagnoses.

Certain embodiments relate to a system and method for classifying and visualizing 3D medical images, with one or more of the following features (and their corresponding advantages): 1) the top-k pooling component enables a 2D CNN to process 3D images, and greatly reduces the number of model parameters, hence training on a small dataset is feasible; 2) a multi-scale CNN feature extractor, which resizes the primary feature maps extracted by a base CNN into different heights and widths, and extracts secondary feature maps that reveal interesting image patterns of different sizes with a shared set of secondary 2D convolutional filters, so that image patterns of different sizes that are most relevant to diagnosis are revealed with a small number of parameters; 3) a data-driven feature reweighting method to offset the differences across different devices and parameter settings, that allows combining training data collected from different devices, and makes the learned model easily generalize to new devices and/or new configurations; 4) due to the flexible processing pipeline, the same model trained using 3D images can be applied to each 2D slice of a 3D image to locate abnormal slices without retraining, enabling a slice-level visualization method to visualize interesting regions within abnormal slices based on gradient back-propagation, to help medical professionals verify the model output and establish reliable diagnosis.

Embodiments may serve as the core component of a Computer-Aided medical image Diagnosis (CAD) system applied to various types of 3D medical images, to assist radiologists in forming quick and reliable diagnosis of relevant pathologies.

At least some embodiments address the three challenges identified above effectively using low computational resources. Although there are other methods attempting to address these challenges as well, they suffer from various limitations, as detailed in the above background section.

Embodiments relate to the automatic classification and visualization of 3D medical images, using a 2D Convolutional Neural Network (CNN). A 2D CNN according to certain embodiments may include a multi-scale 2D CNN feature extractor, a top-k pooling component, a feature reweighting component, and a classifier.

A multi-scale 2D CNN feature extractor may be configured to take a set of 2D images as input, and output a set of feature maps. Each feature map may be a two-dimensional array of float numbers. The feature map may be grouped into a number of convolutional channels, and each channel of the feature map may index one or multiple feature maps. The CNN feature extractor may include multiple convolutional layers, intermediate normalization layers, and intermediate activation layers. In various embodiments, the set of 2D input images may be some or all of the 2D slices in a particular 3D image. The multi-scale CNN feature extractor may include a Base CNN, a set of feature map resizers, and a set of secondary 2D Convolutional filters. The Base CNN may extract primary feature maps, and each of the feature map resizers may resize the feature maps to feature maps of certain height and width. The secondary 2D Convolutional filters may extract interesting image features from the resized feature maps at each scale. In various embodiments, the secondary image features may be grouped by channels, pooled and taken as input by a classifier for diagnosis.

A top-k pooling component may produce a summary feature map as output, given a set of feature maps as input. The top-k pooling component may be configured to take a weighted average of the maximum k values in one or a few coordinates across all feature maps in the input set.

A feature reweighting component may be configured to receive a set of input feature maps and a device identification number (ID), and output a set of transformed feature maps. A feature reweighting component may include multiple sets of affine transformations, each set corresponding to an imaging device. Each set of affine transformations may have the same number of transformations as the number of the convolutional channels of the input feature map, and each affine transformation may correspond to a channel. A feature reweighting component may access the affine transformations corresponding to the input device ID, and transform each channel of the input feature maps with the corresponding parameters of the affine transformations, and then assemble all channels to get the reweighted feature maps.

A classifier may receive a feature map as input, and output a class label. A class label output by a classifier may indicate a most likely class chosen by the classifier, to which the feature maps, as well as the input image that produces these feature maps, may belong. In various embodiments, a classifier may output a confidence value, indicating the certainty of the classifier to the chosen class. In various embodiments, a classifier may be a softmax/logistic regression classifier, a support vector machine, or a gradient boosting model.

A slice-level back-propagation based visualization component may receive a CNN, a convolutional layer identifier referring to a layer in the CNN, an input image and a target class label as input. In various embodiments, the input image may be 3D or 2D. The CNN is able to classify each individual slice within a 3D input image. In one embodiment where the input image is 3D, the CNN may first determine which slices belong to the target class, and for each of these slices, a back-propagation based visualization component may determine the contribution of each pixel to the target class, generate a heatmap that quantifies the contributions, and overlay the heatmap on the 2D slice as the output visualization image. In various embodiments, a visualization component may determine the contribution of each pixel by 1) setting a loss vector of the CNN classifier that encodes the target class, 2) back-propagating the loss to the CNN feature extractor, and 3) extracting the back-propagated gradients at the convolutional layer referred to by the input convolutional layer identifier, summing the gradients over the channel dimension, resizing the sum matrix to the same size as the input image, and using the element in the resized sum matrix as the contribution of the corresponding pixel. In various embodiments, the loss vector of the classifier is set as a vector in which all elements are zero except the dimension of the target class, where the element is set to a negative number. In various embodiments, the back-propagation of gradients are performed using a backward iteration of the CNN model. In various embodiments, after the back-propagation of gradients, the gradients of the parameters of a convolutional layer may be extracted, resized to be the same size as the input image, scaled to be within the interval [0,255], and rounded to an integer. In various embodiments, the resizing of the convolutional layer's gradients may be done using an interpolation algorithm.

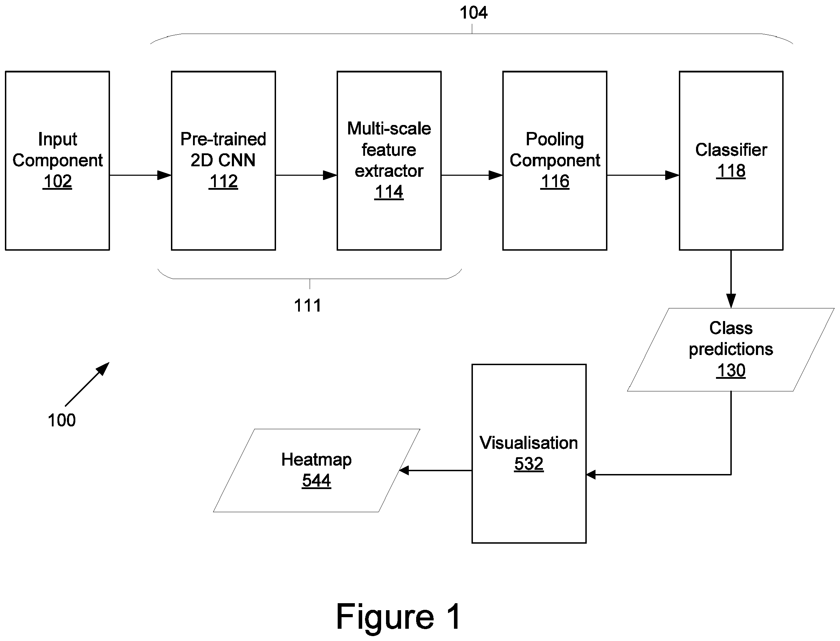

Referring now to Figure 1, a system 100 for classification and visualisation of 3D images may include an input component 102. Input component 102 may obtain a training data set comprising a plurality of training images, which may include 3D images and/or 2D images. Each training image is associated with a class label. Where the training data comprises 3D images, input component 102 may generate 2D images from the 3D images, for example by extracting slices of the 3D images. The same class label may be associated with each 2D image generated from the 3D image.

The input component 102 may obtain the training data set by retrieving previously captured and labelled image data from a local data store, or from a remotely located data store over a network, for example. In some embodiments, the input component 102 may be coupled to an imaging device that captures and stores image data (such as Optical Coherence Tomography data) from a subject that is known to be associated with one or more conditions (such as diabetic retinopathy), such that one or more corresponding class labels can be associated with the captured image(s) *a priori* and provided as training data.

The input component 102 passes the retrieved and/or generated 2D training images to a classification component 104 that comprises a pre-trained 2D CNN 112, also referred to herein as base CNN 112. The base CNN 112 may be an instance of VGG, ResNet, DenseNet or other CNN architectures, with the global pooling and fully-connected layers removed. Examples of CNN architectures useful as a base CNN 112 are described in K. Simonyan, A. Zisserman., "Very Deep Convolutional Networks for Large-Scale Image Recognition", International Conference on Learning Representations, 2015; K. He et al., "Deep Residual Learning for Image Recognition", 2016 IEEE Conference on Computer Vision and Pattern Recognition (CVPR), Las Vegas, NV, 2016, pp. 770-778; and G. Huang et al., "Densely Connected Convolutional Networks", 2017 IEEE Conference on Computer Vision and Pattern Recognition (CVPR), Honolulu, HI, 2017, pp. 2261-2269.

The parameters of the base CNN 112 may be initialised with pre-trained model parameters, or trained from scratch on images collected by the user. In some embodiments, the base CNN 112 may be trained on a large set of natural images, rather than, or in addition to, domain-specific images such as medical images to which the classification system 100 is to be applied.

Given a 3D image as input, the base CNN 112 may treat each slice of the 3D image as an individual 2D image, and extract a set of primary feature maps. The primary feature maps are a 4D tensor the size of which is denoted as $N·C·H·W$, where $N$ is the number of slices, C the number of convolutional channels, and H and W are the height and width of the feature maps. C may depend on the chosen type of base CNN 112; for example, C for ResNet is typically 2048 channels, and for DenseNet, it is typically 1024 channels. In the primary feature maps, some interesting image patterns may have similar feature values, but reside in different sizes of feature blocks (sub-tensors of different heights and/or widths). For a 2D input image, the base CNN 112 may extract a set of primary feature maps of size $C·H·W$.

Base CNN 112 reduces the dimensionality of the input data whilst capturing its correlation structure. In principle, the output of base CNN 112 could be fed directly to a classifier. However, a general purpose CNN may not be suitable for use with medical images, in which regions of interest (ROIs) are often scale-invariant, i.e., visually similar patterns often appear in varying sizes (scales). If approached with general purpose CNNs, an excess number of convolutional kernels with varying receptive fields would be required for full coverage of these patterns, which have more parameters and demand more training data. Some previous works have attempted to learn scale-invariant patterns, for example by adoption of image pyramids, i.e. resizing input images into different scales, processing them with the same CNN and aggregating the outputs. However, it has been found in experiments conducted by the present inventors that image pyramids perform unstably across different datasets and consume much more computational resources than general purpose CNNs.

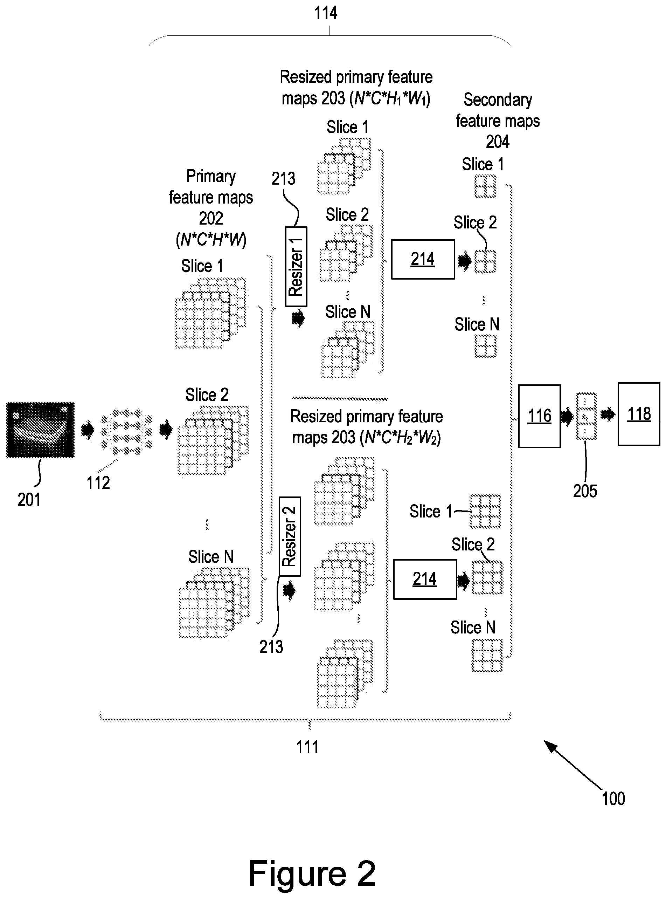

To address the above issue, the system 100 according to certain embodiments passes the primary feature maps 202 output by base CNN 112 to a multi-scale feature extractor 114 that comprises a plurality of feature map resizers 213, as illustrated in Figure 2. For clarity, Figure 2 only shows two different resizing components 213 ("Resizer 1" and "Resizer 2") and one secondary feature channel (the i-th channel). It will be appreciated that additional resizers may be used, for additional feature channels (as well as for the i-th channel), in various embodiments.

The feature map resizers 213 resize the primary feature maps 202 into S different sizes ($N · C · H_1 · W_1, \cdots, N · C · H_S · W_S$). In various embodiments, the resizing algorithm implemented by the respective resizers 213 may be nearest neighbors, bilinear, bicubic or other interpolation algorithms.

More formally, let $x$ denote the primary feature maps, $\{F_1,... ,F_N\}$ denote all the output channels of the multi-scale feature extractor 114, and $\{(h_1,w_1),... , (h_m,w_m)\}$ denote the scale factors of the heights and widths (typically $\frac{1}{4} \le h_i = w_i \le 2$) adopted by the $m$ resizing operators. The combination of the $i$-th scale and the $j$-th channel yields the ij-th secondary feature maps:

$$y_{ij} = F_j \left( \text{Resize}_{h_i,w_i}(x) \right),$$

where in theory Resize$_{hi,wi}$ ($\cdot$) could adopt any type of interpolation as noted above, such as bilinear interpolation.

The multi-scale feature extractor 114 also comprises a plurality of convolutional filters 214 (which may be standard 2D convolutional filters as known to those skilled in the art). For example, as shown in Figure 2, the resized feature maps 203 in each scale may be fed through separate branches to a shared set of secondary 2D convolutional filters 214. The secondary 2D convolutional filters 214 extract secondary feature maps 204 from each group of input resized feature maps 203. Compared to the primary feature maps 202, the secondary feature maps 204 are at a higher-level of abstraction and should therefore be more relevant to the diagnosed pathologies. The secondary 2D convolutional filters 214 have $I$ feature channels, corresponding to $I$ types of interesting patterns. Typically, for medical images, $I$ may be somewhere between 10 and 25. A good choice of $I$ may depend both on the size (number of images) of the training dataset and the diversity of target ROIs. A rule of thumb is that, when there are more training images and/or the target ROIs are more diverse, a larger I is usually better. As the input of secondary 2D convolutional filters 214 comprises multi-scale primary feature maps, the secondary 2D convolutional filters 214 could reveal patterns that are similar, but of different sizes, in the primary feature maps 202, and output the matching degrees of these revealed patterns. Hence each feature channel corresponds to multiple secondary feature maps 204 extracted from the N slices at varied scales.

For more flexibility, the convolutional kernels could also have different kernel sizes. In a setting of $m$ resizing operators and $n$ different sizes of kernels, effectively the kernels have at most $m \times n$ different receptive fields. The multiple resizing operators and varying sizes of kernels complement each other and equip the CNN with scale-invariance.

Pre-trained CNN 112 and multi-scale feature extractor 114 each perform convolutional operations and can be considered collectively to be a convolutional layer or convolutional component 111 of the system 100.

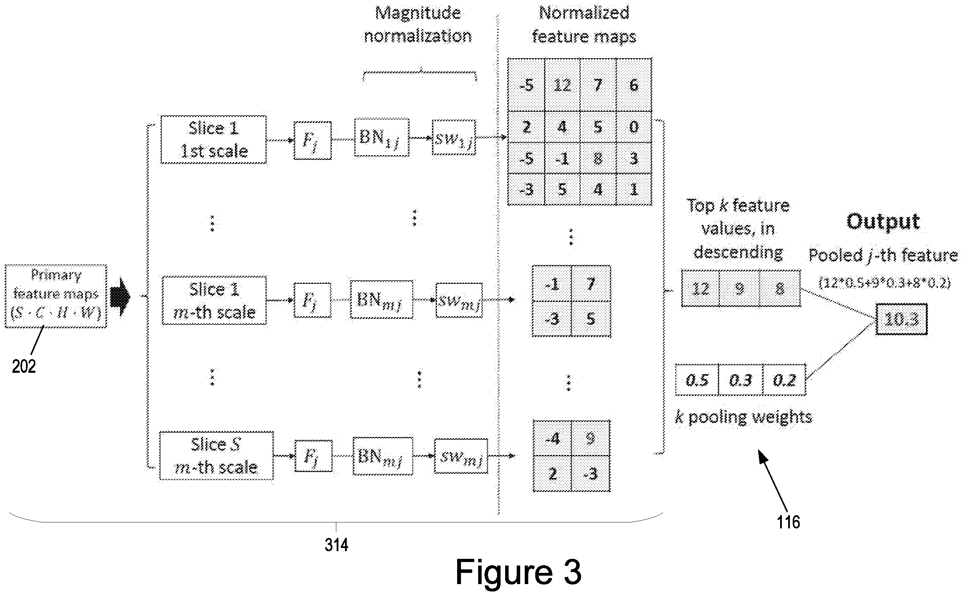

In some embodiments, for example as shown in Figure 3 (which shows operations performed for only the $j$-th channel), feature maps $F_j$ in different scales can be passed through respective magnitude normalization operators. In Figure 3, the multi-scale feature extractor 314 is similar to multi-scale feature extractor 114, except that magnitude normalisation is applied to the primary feature maps 202 prior to pooling. The magnitude normalization operator may comprise a batchnorm operator $BN_{ij}$ and a learnable scalar multiplier $sw_{ij}$. The scalar multiplier $sw_{ij}$ adjusts the importance of the $j$-th channel in the $i$-th scale, and can be optimized by back-propagation.

The system 100 also comprises a pooling component 116 connected to the multi-scale feature extractor 114, that maps the secondary feature maps to a feature vector for input to a classifier 118.

In some embodiments, secondary feature maps of the N input slices (in different heights/widths) belonging to the same feature channel for a 3D input image are pooled into a single feature value by pooling component 116. Hence the $I$ feature channels of the $N$ input slices are pooled into an $I$-dimensional feature vector ($t_1, \cdots, t_I$), which is subsequently taken as input by the classifier 118 to make a prediction (e.g. a diagnosis) for the whole 3D input image.

In other embodiments, secondary feature maps of each slice (in different heights/widths) belonging to the same feature channel for a 3D input image are pooled into a single feature value by pooling component 116. Hence the $I$ feature channels of each input slice are pooled into an $I$-dimensional feature vector ($t_1, \cdots, t_I$), which is subsequently taken as input by the classifier 118 to make a prediction (e.g. a diagnosis) for each individual 2D slice.

The pooling component 116 may comprise a max pooling or average pooling layer. However, the present inventors have found that it is advantageous to use a top-$k$ pooling layer, with $k \geq 2$. An example of the operation of a top-k pooling component 116 is illustrated in Figure 3. In the example of Figure 3, $k=3$ and the pooling component 116 operates on one feature channel (the i-th channel).

In some embodiments, all the $N \cdot J$ feature maps belonging to the $i$-th channel across the N slices are fed to the top-k pooling component 116, which outputs a single pooled value $t_i$. In other embodiments, the $J$ feature maps belonging to the i-th channel of a single slice are fed to the top-k pooling component 116, which outputs a single pooled value $t_i$.

The $I$ feature channels (N $\cdot$ $I$ $\cdot$ $J$ feature maps) are compressed into an $I$-dimensional feature vector ($t_1, \cdots, t_I$). Consequently, the parameters in the downstream classifier are greatly reduced, making it feasible to be trained on small data sets.

The top-k pooling component 116 receives a number of feature maps $X^1, \cdots, X^D$, all of which belong to the i-th channel ({$X^i$} may be of different heights/widths). The largest K numbers within $X^1, \cdots, X^N$ are selected and sorted in descending order, denoted as $x_1, \cdots, x_K$. A top-k pooling component 116 contains $K$ predefined parameters: $K$ weights $w_1, \cdots, w_K$ for the largest $K$ numbers. The weighted sum of the largest $K$ numbers $x_1, \cdots, x_K$, i.e., $t_i = \sum_{k=1}^{K} w_k x_k$ are computed as the output on the i-th channel by the top-k pooling component 116.

In some embodiments, the top-k pooling component 116 may comprise K manually chosen weights, and require no training. In other embodiments, the K weights may be learnable. More formally, given a set of feature maps {$x_i$}, top-k pooling aggregates them into a single value:

$$\mathrm{Pool}_k(\{\boldsymbol{x}_i\}) = \sum_{r=1}^{k} w_r a_r,$$

where $a_1,..., a_k$ are the highest k activations within $\{x_i\}$, and $w_1,..., w_k$ are nonnegative pooling weights to be learned, subject to a normalization constraint $\Sigma_r w_r = 1$. For example, $w_1,..., w_k$ can be initialised with exponentially decayed values, and then optimized with back-propagation.

Although a conventional max-pooling or average-pooling component can also greatly compress features, the top-k pooling component 116 has the following advantages.

- When the model is being optimized using back-propagation (the standard method to train a neural network), for each feature channel, the back-propagated gradients could flow back through the K positions where the K largest numbers reside, and hence K neural pathways are optimized simultaneously during the back propagation. However, in a max-pooling component, only one neural pathway is optimized, which in the case of small training data may increase the network's chance of getting stuck in local minima.

- In an average-pooling component, the largest feature values (corresponding to most salient image patterns) are overwhelmed by many small and uninformative feature values, resulting in lower classification accuracy. In contrast, the top-k pooling always selects the most salient patterns in the input image, and passes them for further diagnosis.

- The top-k pooling is flexible in that it can be used to compress the feature maps of a single slice, or to compress all the feature maps of N slices of a 3D image. Hence a model trained on 3D images can be used to diagnose the same pathologies on 2D images, or vice versa, just by grouping the pooled feature maps in different ways, without any retraining.

To sum up, the top-k pooling component offers faster, more stable and flexible model training and use.

The system 100 also includes a classifier 118 which receives input from the pooling component 116. For example, the classifier 118 may be a softmax/logistic regression classifier, a support vector machine, or a gradient boosting model. Parameters of the multi-scale component 114 and/or the pooling component 116, such as the weights discussed above, can be adjusted in known fashion by backpropagation to minimise the loss function for the classifier and thereby train the CNN 112, 114, 116, 118.

The output of the classification component 104 is one or more class predictions 130. Accordingly, a clinician is provided with an automated assignment of the overall 3D image, or of 2D slices thereof, to one or more classes of clinical significance (said classes being those assigned to images in the training data set).

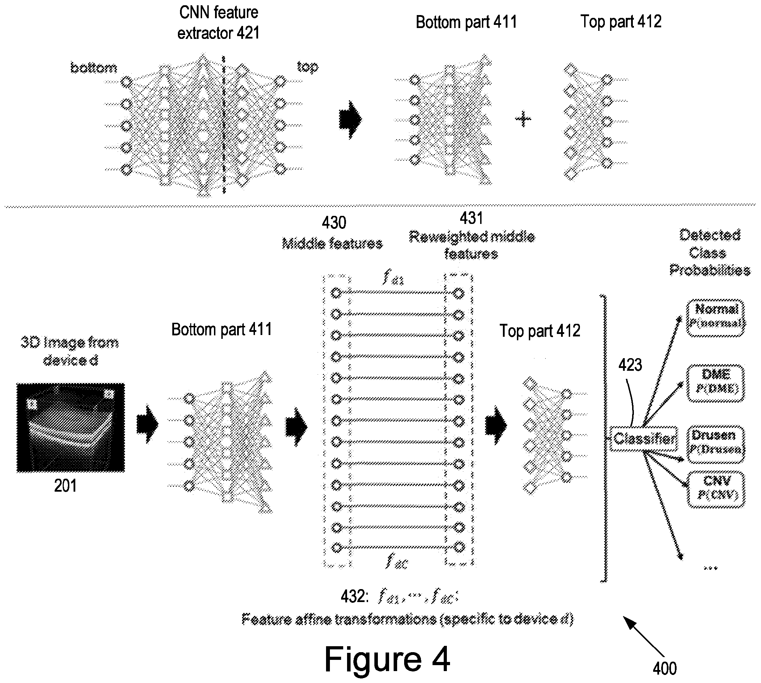

Turning to Figure 4, another embodiment of a classification system 400 includes a feature reweighting component for dealing with images from different devices. System 400 may include a convolutional neural network (CNN) feature extractor 421, which consists of a bottom part 411 and a top part 412, a feature reweighting component 422, and a classifier 423.

In various embodiments, the CNN feature extractor 421 may be VGG, ResNet, DenseNet, the multi-scale CNN feature extractor 114, or other architectures, with the global pooling and fully-connected layers removed. The parameters of the CNN 421 may be pre-trained or trained from scratch on images collected by the user.

The classifier 423 may be a softmax classifier, a neural network, or other classifiers which support computation of gradients or sub-gradients that can be back-propagated from a classification loss function.

The CNN feature extractor 421 is split into two parts: the first $L_1$ layers form the bottom part 411, and the remaining $L_2$ layers form the top part 412. The feature reweighting component 422 sits in between parts 411 and 412. In some embodiments, the feature reweighting component 422 may sit in an intermediate layer of the system 100 of Figure 1, for example in a layer before multi-scale feature extractor 114 and after pre-trained CNN 112, or a layer after the multi-scale feature extractor 114 and before the classifier 118.

Each input image has been captured by a device indexed as d, and there are D devices in total ($D$ is a predefined number). For each input image, the feature reweighting component 432 receives its device ID $d$, and a set of feature vectors or feature maps 430 extracted from the bottom part 411 of the CNN. The set of feature vectors or feature maps 430 are referred to as middle features, which consist of C feature channels.

The feature reweighting component 432 includes $D \cdot C$ feature reweighting affine transformations $f_{11}, \cdots, f_{DC}$. Each affine transformation $f_{di}(x^i) = m_{di}x^i + b_{di}$ is specifically applied to the i-th feature channel of images captured by the d-th device. Each channel $x^i$ of the middle features 430 is transformed by the corresponding affine transformation $f_{di}(x^i)$, yielding transformed middle features 431 of the same size. The transformed middle features 431 are fed into the top part 412 of the CNN feature extractor for further processing, which outputs final features received by a classifier 423 for diagnosis.

In the training stage, all the affine weights $m_{11}, \cdots, m_{DC}$ may be initialized to 1, and all the affine biases $b_{11}, \cdots, b_{DC}$ may be initialized to 0. The affine biases $\{b_{di}\}$ may be trainable or always fixed to be 0. The classifier 423 computes a classification loss function and propagates back loss gradients to optimize the affine parameters $\{m_{di}, b_{di}\}$ (when affine biases are trainable) or $\{m_{di}\}$ (when affine biases are fixed).

Conventionally, device adaptation is usually done with complicated unsupervised learning methods to align the feature distributions of different types of images, such as in Sun et al., "Deep CORAL: Correlation Alignment for Deep Domain Adaptation", In: Hua G., Jégou H. (eds) Computer Vision - ECCV 2016 Workshops, ECCV 2016, Lecture Notes in Computer Science, vol 9915, Springer, Cham. In contrast, the feature reweighting component 432 described here is supervised, in that the affine transformation parameters are tuned by maximizing the classification accuracy on the new type of images, which requires less effort to tune hyperparameters and often finds better transformations to align the two sets of images, given only a small set of images of the new type.

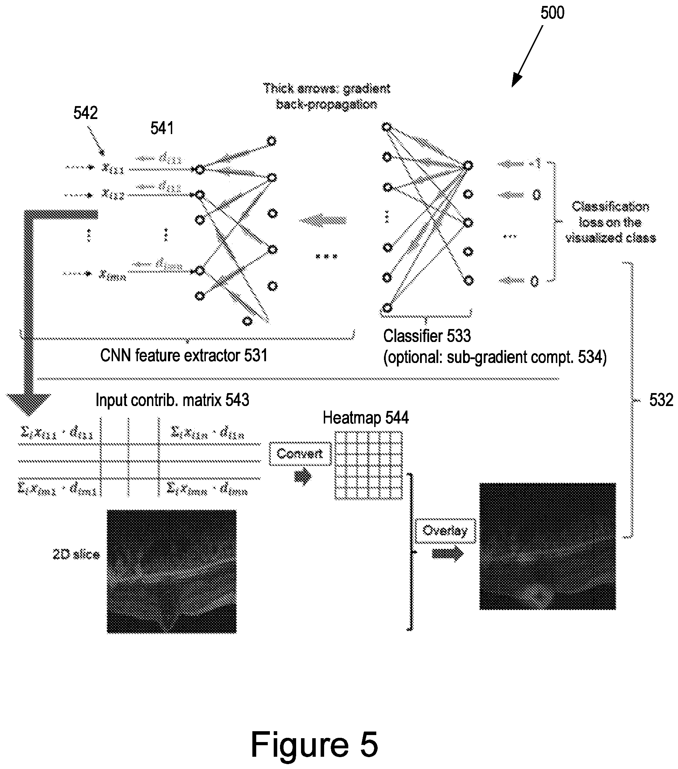

Referring now to Figure 5, an example of a system 500 that includes a back-propagation based visualization component 532 is shown. System 500 may include a CNN feature extractor 531, a heatmap visualization component 532, a classifier 533, and a sub-gradient computation component 534.

In some embodiments, the CNN feature extractor 531 may be the multi-scale CNN feature extractor 114, or another architecture that is able to classify a whole 3D input image, as well as each individual slice within the 3D input image. The parameters of the CNN feature extractor 531 (or 114) may be pre-trained or trained from scratch on images collected by the user. The CNN feature extractor 531 may contain or be connected with a feature reweighting component, such as feature reweighting component 422.

The classifier 533 may be a softmax classifier, a neural network, a logistic regression model, a support vector machine, a gradient boosting model, or other classifiers which support computation of gradients or sub-gradients that can be back-propagated based on a pre-specified classification loss function.

When the classifier 533 does not support direct computation of gradients, an extra sub-gradient computation component 534 may be used (in this or in any other embodiment disclosed herein) to compute sub-gradients with regard to the input of the classifier 533 on a pre-specified classification loss function.

The heatmap visualization component 532 receives an input image, a target class label c (of the classes predicted in class predictions 130) and an index *l* to one of the layers in CNN feature extractor 531. In various embodiments, the input image may be 3D or 2D. In some embodiments where the input image is 3D, the classifier 533 may first determine which slices belong to the target class, and for each of these slices, applies the back-propagation based visualization method to generate a heatmap that quantifies the contributions of each pixel in the input 2D slice, and overlays the heatmap on the 2D slice as the output visualization image. In alternative embodiments where the input image is 2D, the back-propagation based visualization method is directly applied to the 2D image to generate its visualization image.

In various embodiments, the visualization component 532 first fixes the classification loss at the target class c to be a first value (e.g. a negative value such as -1), and the losses of all other classes to be a second value that is different to the first value (such as 0). It then let the classifier 533 propagate back gradients or sub-gradients to CNN feature extractor 531 until the gradients reach the *l*-th layer in CNN feature extractor 531 (the sub-gradients before CNN feature extractor 531 will change to gradients within CNN feature extractor 631).

The heatmap visualization component 532 collects the gradient tensor 541 at the *l*-th layer, denoted as $\{d_{i11}, \cdots, d_{imn}\}$, where *i* indexes the feature channel, and *m,n* indexes the height and width, respectively. The gradient tensor 541 is of the same size as the input feature tensor to the *l*-th layer. The input feature tensor 542 to the *l*-th layer is denoted as $X = \{x_{i11}, \cdots, x_{imn}\}$. Then the heatmap visualization component 532 takes the element-wise multiplication of the gradient tensor 541 and the input feature tensor 542, and sums out the feature channel dimension, so as to get the input contribution matrix 543: $(\Sigma_i d_{11} \cdot x_{11}, \cdots, \Sigma_i d_{mn} \cdot x_{mn})$. By setting all the negative values in the input contribution matrix 543 to 0, scaling to the values into the range [0,255] and rounding to integer values, the input contribution matrix

$$P = \begin{pmatrix} p_{11} & \cdots & p_{1n} \\ \vdots & \ddots & \vdots \\ p_{m1} & \cdots & p_{mn} \end{pmatrix}$$

543 is converted to a non-negative contribution matrix . The non-negative contribution matrix P is interpolated to heatmap 544, denoted as P*, which is of the same size $W_0 \cdot H_0$ as of the original image R. P* is the heatmap that highlights some image areas, and the highlight color intensities are proportional to the contributions of these areas to the final classification confidence of class c. The heatmap visualization component 532 takes a weighted sum of $P*$ and $R$, as the visualization of the basis on which the classifier decides that the input image $R$ is in class c. An instance of such a weighted sum is $0.6 \cdot R + 0.3 \cdot P*$.

There have been a few works on visualizing deep learning classification results, but they are only designed for 2D input images. Previous 3D-CNN models which take 3D images as inputs (even if they can classify the whole 3D image correctly) are unable to determine the contribution of each individual slice, let alone visualize each slice. However, for the convenience of doctors, 2D visualization images are advantageous. The advantage of the presently disclosed slice-level back-propagation based visualization method is that the CNN is flexible and able to classify each individual slice within a 3D input image, and back-propagate only for selected interesting slices for more accurate and targeted visualization.

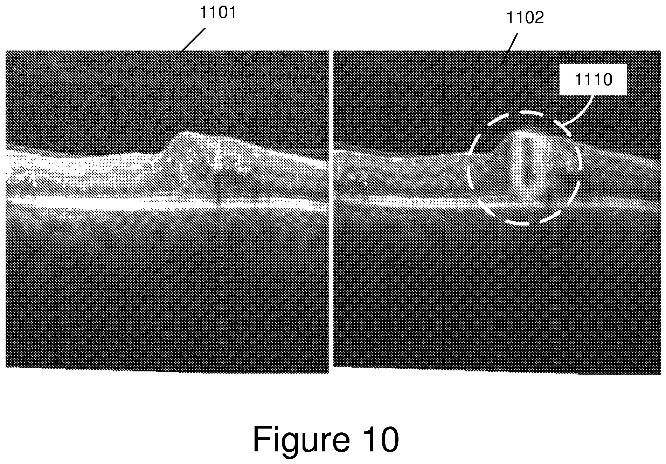

An example of a visualisation generated by system 500 is shown in Figure 10. In Figure 10, a heatmap 1102 overlaid on an OCT slice 1101 is presented. It can be seen that a Diabetic Macular Edema (DME) cyst 1110 is precisely localized in the heatmap 1102 of OCT slice 1101.

As shown in Figure 1, following classification by the classification component 104, the heatmap visualisation component 532 outputs the heatmap 544, which can then be rendered on the display of a computing device. In some embodiments, the computations performed by classification component 104 and heatmap visualisation component 532 can be performed on one or more processors of a single computing system or distributed computing system, and the results of those computations transmitted to a remotely located device, such as a smartphone or other mobile device, for display to a user of the remotely located device. In this way, the computationally expensive operations can be performed by one system, and the results transmitted to users such as clinicians for review.

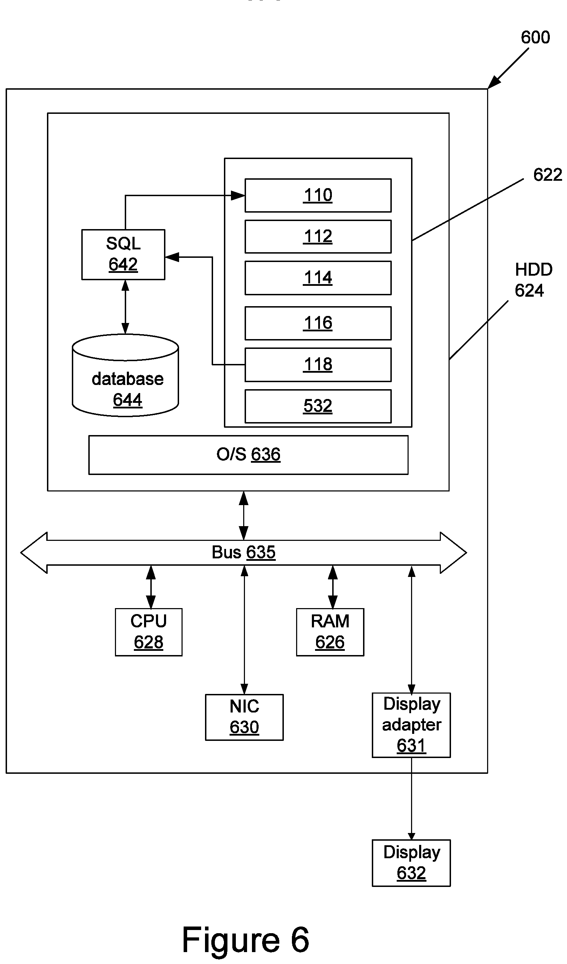

An example of a computing architecture 600 suitable for implementing the system 100 is depicted in Figure 6.

The components of the computing device 600 can be configured in a variety of ways. The components can be implemented entirely by software to be executed on standard computer server hardware, which may comprise one hardware unit or different computer hardware units distributed over various locations, which may communicate over a network. Some of the components or parts thereof may also be implemented by application specific integrated circuits (ASICs) or field programmable gate arrays.

In the example shown in Figure 6, the computing device 600 is a commercially available server computer system based on a 32 bit or a 64 bit Intel architecture, and the processes and/or methods executed or performed by the computing device 600 are implemented in the form of programming instructions of one or more software components or modules 622 stored on non-volatile (e.g., hard disk) computer-readable storage 624 associated with the computing device 600. At least parts of the software modules 622 could alternatively be implemented as one or more dedicated hardware components, such as application-specific integrated circuits (ASICs) and/or field programmable gate arrays (FPGAs).

The computing device 600 includes at least one or more of the following standard, commercially available, computer components, all interconnected by a bus 635:

(a) random access memory (RAM) 626;
(b) at least one computer processor 628;
I a network interface connector (NIC) 630 which connects the computer device 600 to a data communications network and/or to external devices, for example so that training data and/or test data can be received by input component 102, or so that results generated by the classification component (such as class predictions 130) and/or of visualisation component 532 (such as heatmap 544) can be communicated to remotely located users; and
(d) a display adapter 631, which is connected to a display device such as an LCD or LED panel display 632 (in some embodiments, display device 632 may be a touch-screen interface that also enables user input and interaction), for example for displaying class predictions 130 and/or heatmap 544.

The computing device 600 includes a plurality of standard software modules, including:

(a) an operating system (OS) 636 (e.g., Linux or Microsoft Windows);
(b) structured query language (SQL) modules 642 (e.g., MySQL, available from http://www.mysql.com), which allow data, such as class predictions 130, parameters of a trained classification component 104, and/or heatmaps 544, to be stored in and retrieved/accessed from an SQL database 644.

Advantageously, the database 644 forms part of the computer readable data storage 624. Alternatively, the database 644 is located remote from the computing device 600 shown in Figure 6.

The boundaries between the modules and components in the software modules 622 are exemplary, and alternative embodiments may merge modules or impose an alternative decomposition of functionality of modules. For example, the modules 622 discussed herein may be decomposed into submodules to be executed as multiple computer processes, and, optionally, on multiple computers. Moreover, alternative embodiments may combine multiple instances of a particular module or submodule. Furthermore, the operations may be combined or the functionality of the operations may be distributed in additional operations in accordance with the invention. Alternatively, such actions may be embodied in the structure of circuitry that implements such functionality, such as the micro-code of a complex instruction set computer (CISC), firmware programmed into programmable or erasable/programmable devices, the configuration of a field-programmable gate array (FPGA), the design of a gate array or full-custom application-specific integrated circuit (ASIC), or the like.

Each of the parts of the processes performed by the classification system 100 or 400 (such as the process 700) may be executed by a module (of software modules 622) or a portion of a module. The processes may be embodied in a non-transient machine-readable and/or computer-readable medium for configuring a computer system to execute the method. The software modules may be stored within and/or transmitted to a computer system memory to configure the computer system to perform the functions of the module.

The computing device 600 normally processes information according to a program (a list of internally stored instructions such as a particular application program and/or an operating system) and produces resultant output information via input/output (I/O) devices such as NIC 630. A computer process typically includes an executing (running) program or portion of a program, current program values and state information, and the resources used by the operating system to manage the execution of the process. A parent process may spawn other, child processes to help perform the overall functionality of the parent process. Because the parent process specifically spawns the child processes to perform a portion of the overall functionality of the parent process, the functions performed by child processes (and grandchild processes, etc.) may sometimes be described as being performed by the parent process.

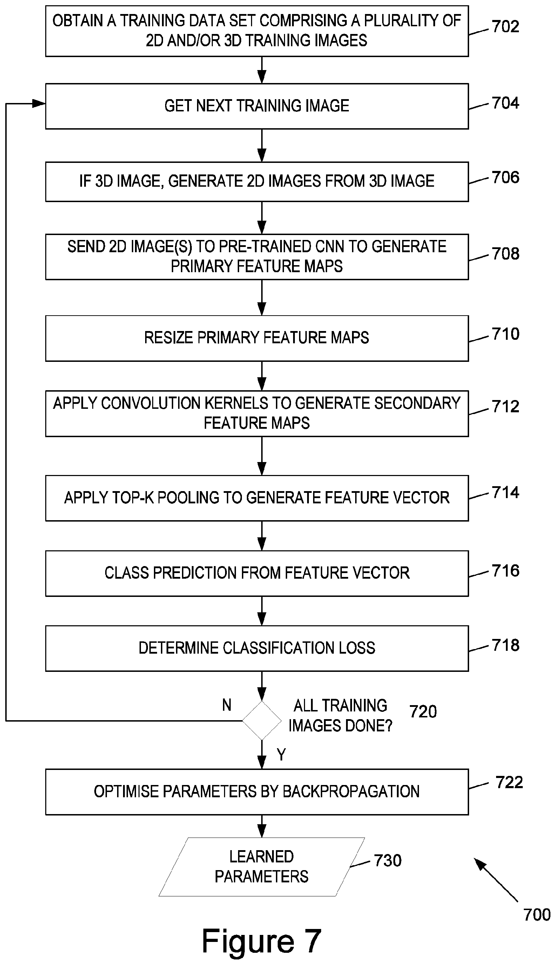

Some steps of an exemplary method 700 for training an image classifier are shown in Figure 7.

At step 702, a training data set comprising a plurality of 2D and/or 3D training images is obtained, for example by input component 110.

At step 704, a first, or the next, training image is obtained from the training data set.

At step 706, the process 700 (e.g., via input component 110) determines whether the training image is a 3D image and if so, generates a plurality of 2D images from the 3D image, for example by taking slices of the 3D image. Each generated 2D image is associated with the overall class label for the 3D image (not shown).

At step 708, the 2D image or images are sent to a pre-trained CNN, such as VGG, ResNet, etc. to generate primary feature maps (e.g., CNN 112 of Figure 1).

At step 710, the primary feature maps are resized (e.g., by resizers 213 of the multi-scale feature extractor 114) to generate resized feature maps.

At step 712, the resized feature maps have a plurality of convolution kernels applied to them to generate secondary feature maps. For example, convolution kernels 214 of multi-scale feature extractor 114 may perform this operation.

At step 714, pooling is applied to the secondary feature maps to generate a feature vector. For example, top-k pooling component 116 may generate the feature vector from the secondary feature maps.

At step 716, a classifier, such as classifier 118, is used to make a class prediction from the generated feature vector.

At step 718, a classification loss is determined, for example based on a difference or differences between predicted class probabilities and the actual classification.

At step 720, the process 700 determines if all training images have been processed. If not, processing returns to step 704. If all images have been processed, then at step 722, parameters of the multi-scale feature extractor 114 and/or pooling component 116 and/or classifier 118 are optimised by backpropagation. The learned parameters 730 output after training can then be used to classify new instances of images passed to the classification system 100. Learned parameters 730 may be stored in a database 644, for example.

**Experimental results**

Experiment 1

An embodiment of the classification system 100 was tested using two different settings.

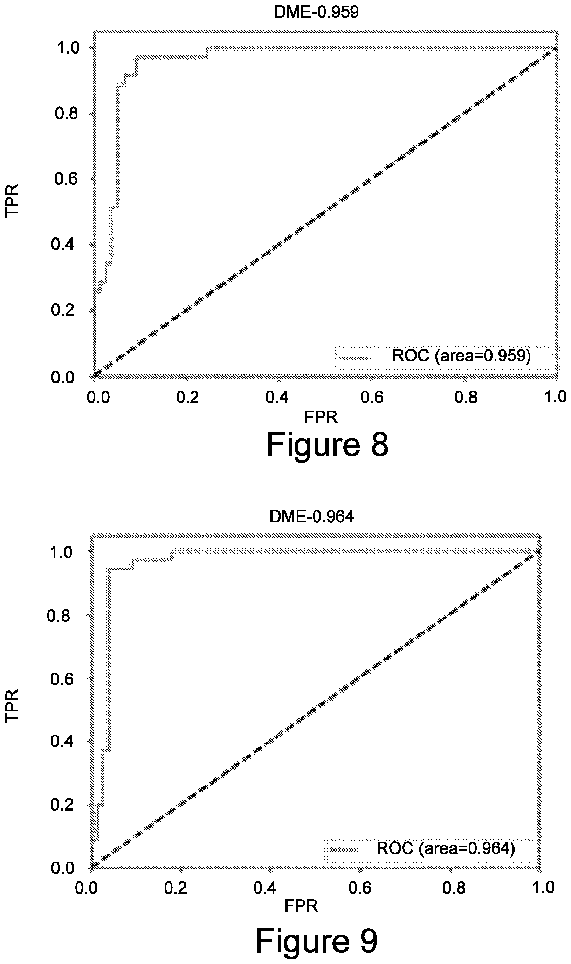

In setting 1, the system 100 was trained on 226 3D-OCT images, and tested on 113 3D-OCT images. Each 3D-OCT image consisted of 128 slices, and each slice had a resolution of 512x128. The classification results evaluated on the doctor-labeled gold standard are shown in Table 1, and Figure 8 shows the ROC curve of the classification results.

**Table 1**

|  | Labeled positive | Labeled negative |
|---|---|---|
| Total | 65 | 161 |
| Classified positive | 61 | 4 |
| Classified negative | 5 | 154 |

(continued)

|  | Labeled positive | Labeled negative |
|---|---|---|
|  | Precision (tp/tp+fp) | Recall (tp/tp+fn) |
|  | 93.8% | 92.4% |

In setting 2, the system 100 was trained on 226 3D OCT images as in setting 1, plus 10,000 2D OCT images. It was tested on the 113 3D-OCT images only. The classification results are shown in Table 2 and the ROC curve is shown in Figure 9. The classification precision, recall and AUC are slightly higher than in setting 1, thanks to the 10,000 2D OCT training images. This illustrates that in order to compensate the shortage of 3D training images, the presently disclosed system 100 is able to take 2D images at the same time to achieve higher accuracy.

**Table 2**

|  | Labeled positive | Labeled negative |
|---|---|---|
| Total | 65 | 161 |
| Classified positive | 61 | 3 |
| Classified negative | 4 | 154 |
|  | Precision (tp/tp+fp) | Recall (tp/tp+fn) |
|  | 95.3% | 93.8% |

Experiment 2

Three classification tasks involving four datasets were used for evaluation.

*DME classification on OCT images.* The following two 3D datasets acquired by Singapore Eye Research Institute (SERI) were used:

1) **Cirrus** dataset: 339 3D OCT images (239 normal, 100 DME). Each image has 128 slices in 512*1024. A 67-33% training/test split was used;

2) **Spectralis** dataset: 197 3D OCT images (60 normal, 137 DME). Each image has 25 _ 31 slices in 497*768. A 50-50% training/test split was used;

*MMD classification on fundus images:*

3) **MMD** dataset (acquired by SERI): 19,272 2D images (11,924 healthy, 631 MMD) in 900*600. A 70-30% training/test split was used.

*MSI/MSS classification on CRC histology images:*

4) **CRC-MSI** dataset (Kather, J.N.: Histological images for MSI vs. MSS classification in gastrointestinal cancer, FFPE samples, https://doi.org/10.5281/zenodo.2530835): 93,408 2D training images (46,704 MSS, 46,704 MSI) in 224*224. 98,904 test images (70,569 MSS, 28,335 MSI) also in 224*224.

MIMS-CNN (system 100), 5 baselines and 6 ablated models were compared. Unless specified, all methods used the ResNet-101 model (without FC) pretrained on ImageNet for feature extraction, and top-k pooling (k = 5) for feature aggregation.

**MI-Pre.** The ResNet feature maps are pooled by top-k pooling and classified.

**Pyramid MI-Pre.** Input images are scaled to $\{\frac{i}{4} | i = 2,3,4\}$ of their original sizes, before being fed into the MI-Pre model.

**MI-Pre-Conv.** The ResNet feature maps are processed by an extra convolutional layer, and aggregated by top-k pooling before classification. It is almost the same as the model in Li et al", "Thoracic disease identification and localization with limited supervis"on", CVPR (June 2018), except that Li et al. does patch-level classification and aggregates patch predictions to obtain image-level classification.

**MIMS (system 100).** The multi-scale feature extractor has 3 resizing operators that resize the primary feature maps to the following scales: $\{\frac{i}{4}|i=2,3,4\}$. Two groups of kernels of different sizes were used.

**MIMS-NoResizing.** It is an ablated version of system 100 with all resizing operators removed. This is to evaluate the contribution of the resizing operators.

**Pyramid MIMS.** It is an ablated version of system 100 with all resizing operators removed, and the multi-scaledness is pursued with input image pyramids of scales $\{\frac{i}{4}|i=2,3,4\}$. The kernels are configured as above.

**MI-Pre-Trident** (Li et al., Scale-Aware Trident Networks for Object Detection. arXiv e-prints arXiv: 1901.01892 (Jan 2019)). It extends MI-Pre-Conv with dilation factors 1, 2, 3.

**SI-CNN.** It is an ablated version of system 100 with the batchnorms $BN_{ij}$ and scalar multipliers $sw_{ij}$ absent from the feature extractor 114.

**FeatPyra-4,5.** It is a feature pyramid network that extracts features from conv4_x and conv5_x in ResNet-101, processes each set of features with a respective convolutional layer, and classifies the aggregate features.

**ResNet34-scratch.** It is a ResNet-34 model trained from scratch.

**MIMS-patchcls** and **MI-Pre-Conv-patchcls.** They are ablated MIMS and MI-Pre-Conv, respectively, evaluated on 3D OCT datasets. They classify each slice, and average slice predictions to obtain image-level classification.

For MIMS-CNN, on the two OCT datasets Cirrus and Spectralis, the convolutional kernels were specified as {2(8), 3(10)} in "kernel size (number of output channels)" pairs. On the MMD dataset, the convolutional kernels were specified as {2(10), 3(10)}. On the CRC-MSI dataset, as the images are of smaller resolution, smaller convolutional kernels {1(20), 2(40)} were adopted.

In all models, the underlying ResNet layers were fine-tuned to reduce domain gaps between ImageNet and the training data. The learning rate of the underlying layers was set as half of the top layers to reduce overfitting.

To evaluate on OCT datasets, all models were first trained on Cirrus for 4500 iterations. Then on Spectralis, the trained models were first fine-tuned on the training set for 200 iterations, then evaluated on the test sets. When training on the Cirrus and Spectralis datasets, to increase data diversity, in each iteration 12-18 slices were randomly chosen to form a batch from the 30 central slices of the input image.

On the CRC-MSI dataset, there is significant domain gap between the training and test images. Hence 2% of the original test images were moved to the training set to reduce the domain gap. In particular, all models were trained on the training set for one epoch (LR=0.01), and then fine-tuned on the tuning set for two epochs (LR=0.01, 0.004).

When working on 3D images such as Cirrus and Spectralis, as the MSConv layer 114 only involves 2D convolutions, all slices in a 3D image can be conveniently arranged as a 2D batch for faster processing.

Performance (in AUROC) of these 12 methods on the above four image datasets is shown in Table 3.

Performance (in AUROC) of seven MIL aggregation schemes on the Cirrus dataset is shown in Table 4.

Table 3 lists the AUROC scores (averaged over three independent runs) of the 12 methods on the four datasets. All methods with an extra convolutional layer on top of a pretrained model performed well. The benefits of using pretrained models are confirmed by the performance gap between ResNet34-scratch and others. The two image pyramid methods performed significantly worse on some datasets, although they consumed twice as much computational time and GPU memory as other methods. MIMS-CNN almost always outperformed other methods.

The inferior performance of the two *-patchcls models demonstrated the advantages of top-k pooling for MIL. To further investigate its effectiveness, we trained MIMS-CNN on Cirrus with six MIL aggregation schemes: average-pooling (mean), max-pooling (max), top-k pooling with k = 2, 3, 4, 5, and an instance-based MIL scheme: max-pooling over slice predictions (max-inst).

As can be seen in Table 4, the other three aggregation schemes fell behind all top-k schemes, and when k increases, the model tends to perform slightly better. It confirms that embedding-based MIL outperforms instance-based MIL.

**Table 3**

| Methods | Cirrus | Spectralis | MMD | CRC-MSI | Avg. |
|---|---|---|---|---|---|
| MI-Pre | 0.574 | 0.906 | 0.956 | 0.880 | 0.829 |
| Pyramid MI-Pre | 0.638 | 0.371 | 0.965 | 0.855 | 0.707 |
| MI-Pre-Conv | 0.972 | 0.990 | 0.961 | 0.870 | 0.948 |
| MIMS-NoResizing | 0.956 | 0.975 | 0.961 | 0.879 | 0.942 |
| Pyramid MIMS | 0.848 | 0.881 | 0.966 | 0.673 | 0.842 |
| MI-Pre-Trident | 0.930 | **1.000** | 0.966 | 0.897 | 0.948 |
| SI-CNN | 0.983 | **1.000** | **0.972** | 0.880 | 0.959 |
| FeatPyra-4,5 | 0.959 | 0.991 | 0.970 | 0.888 | 0.952 |
| ResNet34-scratch | 0.699 | 0.734 | 0.824 | 0.667 | 0.731 |
| MIMS | **0.986** | **1.000** | **0.972** | **0.901** | **0.965** |
| MIMS-patchcls | 0.874 | 0.722 | / | / | / |
| MI-Pre-Conv-patchels | 0.764 | 0.227 | / | / | / |

**Table 4**

| Methods | mean | max | max-inst | $k = 2$ | $k = 3$ | $k = 4$ | $k = 5$ |
|---|---|---|---|---|---|---|---|
| AUROC on Cirrus | 0.829 | 0.960 | 0.975 | 0.980 | 0.980 | 0.986 | 0.986 |

Many modifications will be apparent to those skilled in the art without departing from the scope of the present invention.

Throughout this specification, unless the context requires otherwise, the wo"d "compr"se", and variations such "s "compri"es" a"d "compris"ng", will be understood to imply the inclusion of a stated integer or step or group of integers or steps but not the exclusion of any other integer or step or group of integers or steps.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

3) **MMD** dataset (acquired by SERI): 19,272 2D images (11,924 healthy, 631 MMD) in 900*600. A 70-30% training/test split was used.

*MSI/MSS classification on CRC histology images:*

4) **CRC-MSI** dataset (Kather, J.N.: Histological images for MSI vs. MSS classification in gastrointestinal cancer, FFPE samples, https://doi.org/10.5281/zenodo.2530835): 93,408 2D training images (46,704 MSS, 46,704 MSI) in 224*224. 98,904 test images (70,569 MSS, 28,335 MSI) also in 224*224.

MIMS-CNN (system 100), 5 baselines and 6 ablated models were compared. Unless specified, all methods used the ResNet-101 model (without FC) pretrained on ImageNet for feature extraction, and top-k pooling (k = 5) for feature aggregation.

**MI-Pre.** The ResNet feature maps are pooled by top-k pooling and classified.

**Pyramid MI-Pre.** Input images are scaled to $\{\frac{i}{4}|i = 2,3,4\}$ of their original sizes, before being fed into the MI-Pre model.

**MI-Pre-Conv.** The ResNet feature maps are processed by an extra convolutional layer, and aggregated by top-k pooling before classification. It is almost the same as the model in Li et al., "Thoracic disease identification and localization with limited supervision", CVPR (June 2018), except that Li et al. does patch-level classification and aggregates patch predictions to obtain image-level classification.

**MIMS (system 100).** The multi-scale feature extractor has 3 resizing operators that resize the primary feature maps to the following scales: $\{\frac{i}{4}|i=2,3,4\}$ . Two groups of kernels of different sizes were used.

**MIMS-NoResizing.** It is an ablated version of system 100 with all resizing operators removed. This is to evaluate the contribution of the resizing operators.

**Pyramid MIMS.** It is an ablated version of system 100 with all resizing operators removed, and the multi-scaledness is pursued with input image pyramids of scales $\{\frac{i}{4}|i=2,3,4\}$ . The kernels are configured as above.

**MI-Pre-Trident** (Li et al., Scale-Aware Trident Networks for Object Detection. arXiv e-prints arXiv: 1901.01892 (Jan 2019)). It extends MI-Pre-Conv with dilation factors 1, 2, 3.

**SI-CNN.** It is an ablated version of system 100 with the batchnorms $BN_{ij}$ and scalar multipliers $sw_{ij}$ absent from the feature extractor 114.

**FeatPyra-4,5.** It is a feature pyramid network that extracts features from conv4_x and conv5_x in ResNet-101, processes each set of features with a respective convolutional layer, and classifies the aggregate features.

**ResNet34-scratch.** It is a ResNet-34 model trained from scratch.

**MIMS-patchcls** and **MI-Pre-Conv-patchcls.** They are ablated MIMS and MI-Pre-Conv, respectively, evaluated on 3D OCT datasets. They classify each slice, and average slice predictions to obtain image-level classification.

For MIMS-CNN, on the two OCT datasets Cirrus and Spectralis, the convolutional kernels were specified as {2(8), 3(10)} in "kernel size (number of output channels)" pairs. On the MMD dataset, the convolutional kernels were specified as {2(10), 3(10)}. On the CRC-MSI dataset, as the images are of smaller resolution, smaller convolutional kernels {1(20), 2(40)} were adopted.

In all models, the underlying ResNet layers were fine-tuned to reduce domain gaps between ImageNet and the training data. The learning rate of the underlying layers was set as half of the top layers to reduce overfitting.

To evaluate on OCT datasets, all models were first trained on Cirrus for 4500 iterations. Then on Spectralis, the trained models were first fine-tuned on the training set for 200 iterations, then evaluated on the test sets. When training on the Cirrus and Spectralis datasets, to increase data diversity, in each iteration 12-18 slices were randomly chosen to form a batch from the 30 central slices of the input image.

On the CRC-MSI dataset, there is significant domain gap between the training and test images. Hence 2% of the original test images were moved to the training set to reduce the domain gap. In particular, all models were trained on the training set for one epoch (LR=0.01), and then fine-tuned on the tuning set for two epochs (LR=0.01, 0.004).

When working on 3D images such as Cirrus and Spectralis, as the MSConv layer 114 only involves 2D convolutions, all slices in a 3D image can be conveniently arranged as a 2D batch for faster processing.

Performance (in AUROC) of these 12 methods on the above four image datasets is shown in Table 3.

Performance (in AUROC) of seven MIL aggregation schemes on the Cirrus dataset is shown in Table 4.

Table 3 lists the AUROC scores (averaged over three independent runs) of the 12 methods on the four datasets. All methods with an extra convolutional layer on top of a pretrained model performed well. The benefits of using pretrained models are confirmed by the performance gap between ResNet34-scratch and others. The two image pyramid methods performed significantly worse on some datasets, although they consumed twice as much computational time and GPU memory as other methods. MIMS-CNN almost always outperformed other methods.

The inferior performance of the two *-patchcls models demonstrated the advantages of top-k pooling for MIL. To further investigate its effectiveness, we trained MIMS-CNN on Cirrus with six MIL aggregation schemes: average-pooling (mean), max-pooling (max), top-k pooling with k = 2, 3, 4, 5, and an instance-based MIL scheme: max-pooling over slice predictions (max-inst).

As can be seen in Table 4, the other three aggregation schemes fell behind all top-k schemes, and when k increases, the model tends to perform slightly better. It confirms that embedding-based MIL outperforms instance-based MIL.

**Table 3**

| Methods | Cirrus | Spectralis | MMD | CRC-MSI | Avg. |
|---|---|---|---|---|---|
| MI-Pre | 0.574 | 0.906 | 0.956 | 0.880 | 0.829 |
| Pyramid MI-Pre | 0.638 | 0.371 | 0.965 | 0.855 | 0.707 |
| MI-Pre-Conv | 0.972 | 0.990 | 0.961 | 0.870 | 0.948 |
| MIMS-NoResizing | 0.956 | 0.975 | 0.961 | 0.879 | 0.942 |
| Pyramid MIMS | 0.848 | 0.881 | 0.966 | 0.673 | 0.842 |
| MI-Pre-Trident | 0.930 | **1.000** | 0.966 | 0.897 | 0.948 |

(continued)

| Methods | Cirrus | Spectralis | MMD | CRC-MSI | Avg. |
|---|---|---|---|---|---|
| SI-CNN | 0.983 | **1.000** | **0.972** | 0.880 | 0.959 |
| FeatPyra-4,5 | 0.959 | 0.991 | 0.970 | 0.888 | 0.952 |
| ResNet34-scratch | 0.699 | 0.734 | 0.824 | 0.667 | 0.731 |
| MIMS | **0.986** | **1.000** | **0.972** | **0.901** | **0.965** |
| MIMS-patchels | 0.874 | 0.722 | / | / | / |
| MI-Pre-Conv-patchels | 0.764 | 0.227 | / | / | / |

**Table 4**

| Methods | mean | max | max-inst | $k = 2$ | $k = 3$ | $k = 4$ | $k = 5$ |
|---|---|---|---|---|---|---|---|
| AUROC on Cirrus | 0.829 | 0.960 | 0.975 | 0.980 | 0.980 | 0.986 | 0.986 |

Many modifications will be apparent to those skilled in the art without departing from the scope of the present invention.

Throughout this specification, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

**Claims**

1. A computer-aided diagnosis (CAD) system for classification and visualisation of a 3D medical image, comprising:

   a classification component (104) comprising a 2D convolutional neural network (CNN) that is configured to generate a prediction of one or more classes for 2D slices of the 3D medical image; and
   a visualisation component (532) that is configured to:

   determine, for a target class of said one or more classes, which slices belong to the target class;
   **characterised in that**, for each identified slice, the visualisation component (532) is configured to determine a relative contribution of each pixel of the identified slice to classification of the identified slice as belonging to the target class by:

   setting a classification loss for the target class to be a first value, and for all other classes to be a second value that is different to the first value;
   computing, from the classification loss, error gradients;
   backpropagating the error gradients to an intermediate layer of the 2D CNN; and
   determining, from a gradient tensor at the predetermined intermediate layer, an input contribution matrix representing the relative contributions of the pixels of the 2D slice to a class probability of the target class; and
   generating, from the input combination matrix, a heatmap that provides a visual indication of the contributions of respective pixels, and **in that** the 2D CNN comprises:

   a first convolutional neural network (CNN) component configured to extract a set of primary feature maps from 2D slices of the 3D image;
   a multi-scale feature extractor (114) configured to generate a set of secondary feature maps from the primary feature maps, wherein the multi-scale feature extractor comprises:

a plurality of resizers (213), respective resizers (213) being configured to generate respective resized feature maps, each resizer (213) being **characterised by** a different size parameter; and

a plurality of convolution filters configured to generate the secondary feature maps from the resized feature maps;

a pooling component (116) configured to generate a feature vector from the secondary feature maps; and

a classifier (118) configured to generate one or more class predictions for the 3D image based on the feature vector;

wherein the pooling component (116) comprises a top-k pooling layer that is configured to:

determine top k values across the secondary feature maps for each of a plurality of convolutional channels, where $k >= 2$; and

compute a weighted average of the top k values.

2.  A CAD system according to claim 1, wherein the visualisation component (532) is further configured to cause a display to render the heatmap as an overlay on the identified slice.

3.  A CAD system according to claim 1, wherein the classification component (104) is configured to apply the same set of convolution filters to resized feature maps having different respective size parameters.

4.  A CAD system according to any one of the preceding claims, wherein the CNN is configured to perform a device adaptation operation in accordance with a device identifier of a device by which the 3D image was captured; wherein the device adaptation operation comprises obtaining parameters of at least one affine transformation for said device, affine parameters of the affine transformations being optimized by backpropagation of loss gradients; and applying the at least one affine transformation to reweight features in an intermediate layer of the CNN.

5.  A CAD system according to any one of the preceding claims, further comprising a training component that is configured to:

obtain a training data set comprising a plurality of 3D training images, each 3D training image having associated therewith an overall class label;

for each 3D training image:

generate a plurality of 2D input images from the 3D training image, each 2D input image being assigned the overall class label;

pass the 2D input images to the CNN; and

applying backpropagation to a loss function for a classifier of the CNN to thereby train the CNN;

optionally wherein the training data set further comprises 2D training images, and wherein the 2D training images are passed to the CNN to generate class predictions for the 2D training images.

6.  A computer- implemented, computer-aided diagnosis (CAD) method, comprising:

receiving a 3D medical image;

generating a prediction of one or more classes for 2D slices of the 3D medical image using a 2D convolutional neural network (CNN);

determining, for a target class of said one or more classes, which slices belong to the target class;

**characterised in that**, for each identified slice, the method comprises determining, a contribution of each pixel of the identified slice to classification of the identified slice as belonging to the target class by:

setting a classification loss for the target class to be a first value, and for all other classes to be a second value that is different to the first value;

computing, from the classification loss, error gradients;

backpropagating the error gradients to the intermediate layer of the 2D CNN; and

determining, from the gradient tensor at the predetermined intermediate layer, an input contribution matrix representing the relative contributions of respective regions of the 2D slice to the class probability of the target class; and

generating a heatmap, from the input contribution matrix, that provides a visual indication of the contributions of respective pixels, and **in that** the 2D CNN comprises:

a first convolutional neural network (CNN) component configured to extract a set of primary feature maps from 2D slices of the 3D image;
a multi-scale feature extractor (114) configured to generate a set of secondary feature maps from the primary feature maps, wherein the multi-scale feature extractor comprises:

a plurality of resizers (213), respective resizers (213) being configured to generate respective resized feature maps, each resizer (213) being **characterised by** a different size parameter; and
a plurality of convolution filters configured to generate the secondary feature maps from the resized feature maps;

a pooling component (116) configured to generate a feature vector from the secondary feature maps; and
a classifier (118) configured to generate one or more class predictions for the 3D image based on the feature vector;
wherein the pooling component (116) comprises a top-k pooling layer that is configured to:

determine top k values across the secondary feature maps for each of a plurality of convolutional channels, where k >= 2; and
compute a weighted average of the top k values.

7. A CAD method according to claim 6, further comprising causing a display to render the heatmap as an overlay on the identified slice.

8. A CAD method according to claim 6 or 7, wherein the classification component (104) is configured to apply the same set of convolution filters to resized feature maps having different respective size parameters.

9. A CAD method according to any one of claims 6 to 8, wherein the CNN is configured to perform a device adaptation operation in accordance with a device identifier of a device by which the 3D image was captured, wherein the device adaptation operation comprises obtaining parameters of at least one affine transformation for said device, affine parameters of the affine transformations being optimized by backpropagation of loss gradients; and applying the at least one affine transformation to reweight features in an intermediate layer of the CNN.

10. A CAD method according to any one of claims 6 to 9, further comprising training the CNN by:

obtaining a training data set comprising a plurality of 3D training images, each 3D training image having associated therewith an overall class label;
for each 3D training image:

generating a plurality of 2D input images from the 3D training image, each 2D input image being assigned the overall class label;
passing the 2D input images to the CNN; and

applying backpropagation to a loss function for a classifier of the CNN to thereby train the CNN;
optionally wherein the training data set further comprises 2D training images, and wherein the 2D training images are passed to the CNN to generate class predictions for the 2D training images.

11. A non-volatile computer-readable storage medium having instructions stored thereon for causing at least one processor (628) to perform a method according to any one of claims 6 to 10.

**Patentansprüche**

1. Computerunterstütztes Diagnose (CAD) -System zur Klassifizierung und Visualisierung eines medizinischen 3D-Bildes, umfassend:

eine Klassifizierungskomponente (104), umfassend ein neuronales 2D-Faltungsnetzwerk (CNN), das ausgelegt ist, um eine Vorhersage einer oder mehrerer Klassen für 2D-Schnitte des medizinischen 3D-Bildes zu erzeugen; und

eine Visualisierungskomponente (532), die ausgelegt ist zum:

Bestimmen, für eine Zielklasse der einen oder mehreren Klassen, welche Schnitte zur Zielklasse gehören; **dadurch gekennzeichnet, dass** die Visualisierungskomponente (532), für jeden identifizierten Schnitt, ausgelegt ist, um einen relativen Betrag jedes Pixels des identifizierten Schnitts zur Klassifizierung des identifizierten Schnitts als zur Zielklasse gehörend zu bestimmen durch:

Festlegen eines Klassifizierungsverlustes für die Zielklasse als ersten Wert und für alle andere Klassen als zweiten Wert, der sich vom ersten Wert unterscheidet;
Berechnen von Fehlergradienten aus dem Klassifizierungsverlust;
Rückwärtspropagieren der Fehlergradienten auf eine Zwischenschicht des 2D-CNN; und
Bestimmen, aus einem Gradiententensor an der vorbestimmten Zwischenschicht, einer Eingabebeitragsmatrix, die die relativen Beträge der Pixel des 2D-Schnitts zu einer Klassenwahrscheinlichkeit der Zielklasse darstellt; und
Erzeugen, aus der Eingabekombinationsmatrix, einer Heatmap, die eine visuelle Angabe der Beiträge jeweiliger Pixel bereitstellt, und dadurch, dass das 2D-CNN umfasst:

eine erste Komponente des neuronalen Faltungsnetzwerks (CNN), die ausgelegt ist, um einen Satz von Primärmerkmalskarten aus 2D-Schnitten des 3D-Bildes zu extrahieren;
einen Mehrfachskalenmerkmalsextraktor (114), der ausgelegt ist, um einen Satz von Sekundärmerkmalskarten aus den Primärmerkmalskarten zu erzeugen, wobei der Mehrfachskalenmerkmalsextraktor umfasst:

eine Vielzahl von Größenanpassern (213), wobei jeweilige Größenanpasser (213) ausgelegt sind, um jeweilige größenangepasste Merkmalskarten zu erzeugen, wobei jeder Größenanpasser (213) durch einen unterschiedlichen Größenparameter gekennzeichnet ist, und
eine Vielzahl von Konvolutionsfiltern, die ausgelegt sind, um die Sekundärmerkmalskarten aus den größenangepassten Merkmalskarten zu erzeugen;

eine Pooling-Komponente (116), die ausgelegt ist, um einen Merkmalsvektor aus den Sekundärmerkmalskarten zu erzeugen; und
einen Klassifikator (118), der konfiguriert ist, um eine oder mehrere Klassenvorhersagen für das 3D-Bild auf Grundlage des Merkmalsvektors zu erzeugen;
wobei die Pooling-Komponente (116) eine Pooling-Schicht der obersten k umfasst, die ausgelegt ist zum:

Bestimmen von obersten k Werten über die Sekundärmerkmalskarten für jeden einer Vielzahl von Konvolutionskanälen, wobei $k >= 2$; und
Berechnen eines gewichteten Durchschnitts der obersten k Werte.

2. CAD-System nach Anspruch 1, wobei die Visualisierungskomponente (532) weiter ausgelegt ist, um zu veranlassen, dass eine Anzeige die Heatmap als eine Überlagerung auf dem identifizierten Schnitt wiedergibt.

3. CAD-System nach Anspruch 1, wobei die Klassifizierungskomponente (104) ausgelegt ist, um den gleichen Satz von Konvolutionsfiltern an größenangepassten Merkmalskarten anzuwenden, die unterschiedliche jeweilige Größenparameter aufweisen.

4. CAD-System nach einem der vorstehenden Ansprüche, wobei das CNN ausgelegt ist, um einen Vorrichtungseinstellungsvorgang gemäß einer Vorrichtungskennung einer Vorrichtung durchzuführen, mit der das 3D-Bild aufgenommen wurde; wobei der Vorrichtungseinstellungsvorgang umfasst, Parameter von mindestens einer affinen Transformation für die Vorrichtung zu erhalten, wobei affine Parameter der affinen Transformationen durch Rückwärtspropagation von Verlustgradienten optimiert werden; und die mindestens eine affine Transformation anzuwenden, um Merkmale in einer Zwischenschicht des CNN neu zu gewichten.

5. CAD-System nach einem der vorstehenden Ansprüche, weiter umfassend eine Trainingskomponente, die ausgelegt ist zum:

Erhalten eines Trainingsdatensatzes, der eine Vielzahl von 3D-Trainingsbildern umfasst, wobei jedem 3D-Trainingsbild eine Gesamtklassenbezeichnung zugeordnet ist;
für jedes 3D-Trainingsbild:

Erzeugen einer Vielzahl von 2D-Eingabebildern aus dem 3D-Trainingsbild, wobei jedes 2D-Eingabebild der Gesamtklassenbezeichnung zugewiesen ist;
Weitergeben der 2D-Eingabebilder an das CNN; und

Anwenden von Rückwärtspropagation an eine Verlustfunktion für einen Klassifikator des CNN, um dadurch das CNN zu trainieren;
wobei der Trainingsdatensatz wahlweise weiter 2D-Trainingsbilder umfasst und wobei die 2D-Trainingsbilder an das CNN weitergegeben werden, um Klassenvorhersagen für die 2D-Trainingsbilder zu erzeugen.

6. Computerimplementiertes computerunterstütztes Diagnose (CAD) -Verfahren, umfassend:

Empfangen eines medizinischen 3D-Bildes,
Erzeugen einer Vorhersage einer oder mehrerer Klassen für 2D-Schnitte des medizinischen 3D-Bildes unter Verwendung eines neuronalen 2D-Faltungsnetzwerks (CNN);
Bestimmen, für eine Zielklasse der einen oder mehreren Klassen, welche Schnitte zur Zielklasse gehören;
**dadurch gekennzeichnet, dass** das Verfahren für jeden identifizierten Schnitt umfasst, einen Betrag jedes Pixels des identifizierten Schnitts zur Klassifizierung des identifizierten Schnitts als zur Zielklasse gehörend zu bestimmen durch:

Festlegen eines Klassifizierungsverlustes für die Zielklasse als ersten Wert und für alle andere Klassen als zweiten Wert, der sich vom ersten Wert unterscheidet;
Berechnen von Fehlergradienten aus dem Klassifizierungsverlust;
Rückwärtspropagieren der Fehlergradienten auf die Zwischenschicht des 2D-CNN; und
Bestimmen, aus dem Gradiententensor an der vorbestimmten Zwischenschicht, einer Eingabebeitragsmatrix, die die relativen Beträge von jeweiligen Bereichen des 2D-Schnitts zur Klassenwahrscheinlichkeit der Zielklasse darstellt; und

Erzeugen einer Heatmap aus der Eingabebeitragsmatrix, die eine visuelle Angabe der Beiträge jeweiliger Pixel bereitstellt, und dadurch, dass das 2D-CNN umfasst:

eine erste Komponente des neuronalen Faltungsnetzwerks (CNN), die ausgelegt ist, um einen Satz von Primärmerkmalskarten aus 2D-Schnitten des 3D-Bildes zu extrahieren;
einen Mehrfachskalenmerkmalsextraktor (114), der ausgelegt ist, um einen Satz von Sekundärmerkmalskarten aus den Primärmerkmalskarten zu erzeugen, wobei der Mehrfachskalenmerkmalsextraktor umfasst:

eine Vielzahl von Größenanpassern (213), wobei jeweilige Größenanpasser (213) ausgelegt sind, um jeweilige größenangepasste Merkmalskarten zu erzeugen, wobei jeder Größenanpasser (213) durch einen unterschiedlichen Größenparameter gekennzeichnet ist, und
eine Vielzahl von Konvolutionsfiltern, die ausgelegt sind, um die Sekundärmerkmalskarten aus den größenangepassten Merkmalskarten zu erzeugen;

eine Pooling-Komponente (116), die ausgelegt ist, um einen Merkmalsvektor aus den Sekundärmerkmalskarten zu erzeugen; und
einen Klassifikator (118), der konfiguriert ist, um eine oder mehrere Klassenvorhersagen für das 3D-Bild auf Grundlage des Merkmalsvektors zu erzeugen;
wobei die Pooling-Komponente (116) eine Pooling-Schicht der obersten k umfasst, die ausgelegt ist zum:

Bestimmen von obersten k Werten über die Sekundärmerkmalskarten für jeden der Vielzahl von Konvolutionskanälen, wobei k >= 2; und
Berechnen eines gewichteten Durchschnitts der obersten k Werte.

7. CAD-Verfahren nach Anspruch 6, weiter umfassend Veranlassen, dass eine Anzeige die Heatmap als eine Überlagerung auf dem identifizierten Schnitt wiedergibt.

8. CAD-Verfahren nach Anspruch 6 oder 7, wobei die Klassifizierungskomponente (104) ausgelegt ist, um den gleichen Satz von Konvolutionsfiltern an größenangepassten Merkmalskarten anzuwenden, die unterschiedliche jeweilige Größenparameter aufweisen.

9. CAD-Verfahren nach einem der Ansprüche 6 bis 8, wobei das CNN ausgelegt ist, um einen Vorrichtungseinstellungsvorgang gemäß einer Vorrichtungskennung einer Vorrichtung durchzuführen, mit der das 3D-Bild aufgenommen wurde, wobei der Vorrichtungseinstellungsvorgang umfasst, Parameter von mindestens einer affinen Transformation für die Vorrichtung zu erhalten, wobei affine Parameter der affinen Transformationen durch Rückwärtssropagation von Verlustgradienten optimiert werden; und die mindestens eine affine Transformation anzuwenden, um Merkmale in einer Zwischenschicht des CNN neu zu gewichten.

10. CAD-Verfahren nach einem der Ansprüche 6 bis 9, weiter umfassend Trainieren des CNN durch:

Erhalten eines Trainingsdatensatzes, der eine Vielzahl von 3D-Trainingsbildern umfasst, wobei jedem 3D-Trainingsbild eine Gesamtklassenbezeichnung zugeordnet ist;
für jedes 3D-Trainingsbild:

Erzeugen einer Vielzahl von 2D-Eingabebildern aus dem 3D-Trainingsbild, wobei jedes 2D-Eingabebild der Gesamtklassenbezeichnung zugewiesen ist;
Weitergeben der 2D-Eingabebilder an das CNN; und

Anwenden von Rückwärtspropagation an eine Verlustfunktion für einen Klassifikator des CNN, um dadurch das CNN zu trainieren;
wobei der Trainingsdatensatz wahlweise weiter 2D-Trainingsbilder umfasst und wobei die 2D-Trainingsbilder an das CNN weitergegeben werden, um Klassenvorhersagen für die 2D-Trainingsbilder zu erzeugen.

11. Nichtflüchtiges computerlesbares Speichermedium, auf dem Anweisungen gespeichert sind, die veranlassen, dass der mindestens eine Prozessor (628) ein Verfahren nach einem der Ansprüche 6 bis 10 durchführt.


**Revendications**

1. Système de diagnostic assisté par ordinateur (DAO) pour la classification et la visualisation d'une image médicale 3D, comprenant :

un composant de classification (104) comprenant un réseau neuronal convolutif (CNN) 2D qui est configuré pour générer une prédiction d'une ou plusieurs classes pour des coupes 2D de l'image médicale 3D ; et
un composant de visualisation (532) qui est configuré pour :

déterminer, pour une classe cible desdites une ou plusieurs classes, les tranches qui appartiennent à la classe cible ;
**caractérisé en ce que**, pour chaque tranche identifiée, le composant de visualisation (532) est configuré pour déterminer une contribution relative de chaque pixel de la tranche identifiée à la classification de la tranche identifiée comme appartenant à la classe cible par :

la définition d'une perte de classification pour la classe cible à une première valeur, et pour toutes les autres classes à une seconde valeur qui est différente de la première valeur ;
le calcul, à partir de la perte de classification, de gradients d'erreur ;
la rétropropagation des gradients d'erreur vers une couche intermédiaire du CNN 2D ; et
la détermination, à partir d'un tenseur de gradient à la couche intermédiaire prédéterminée, d'une matrice de contribution d'entrée représentant les contributions relatives des pixels de la tranche 2D à une probabilité de classe de la classe cible ; et
la génération, à partir de la matrice de combinaison d'entrée, d'une carte thermique qui fournit une indication visuelle des contributions de pixels respectifs, et **en ce que** le CNN 2D comprend :

un premier composant de réseau neuronal convolutif (CNN) configuré pour extraire un ensemble de cartes de caractéristiques primaires à partir de tranches 2D de l'image 3D;
un extracteur de caractéristiques multiéchelle (114) configuré pour générer un ensemble de cartes de caractéristiques secondaires à partir des cartes de caractéristiques primaires, dans lequel l'extracteur de caractéristiques multiéchelle comprend :

une pluralité de redimensionneurs (213), les redimensionneurs respectifs (213) étant configurés pour générer des cartes de caractéristiques redimensionnées respectives, chaque redimensionneur (213) étant **caractérisé par** un paramètre de taille différent ; et
une pluralité de filtres de convolution configurés pour générer les cartes de caractéristiques secondaires à partir des cartes de caractéristiques redimensionnées ;

un composant de mise en commun (116) configuré pour générer un vecteur de caractéristiques à partir des cartes de caractéristiques secondaires ; et
un classificateur (118) configuré pour générer une ou plusieurs prédictions de classe pour l'image 3D sur la base du vecteur de caractéristiques ;
dans lequel le composant de mise en commun (116) comprend une couche de mise en commun de type top-k qui est configurée pour :

déterminer les k premières valeurs des cartes de caractéristiques secondaires pour chacun d'une pluralité de canaux convolutifs, où $k >= 2$ ; et
calculer une moyenne pondérée des k premières valeurs.

2. Système de DAO selon la revendication 1, dans lequel le composant de visualisation (532) est en outre configuré pour amener un écran à restituer la carte thermique sous forme de superposition sur la tranche identifiée.

3. Système de DAO selon la revendication 1, dans lequel le composant de classification (104) est configuré pour appliquer le même ensemble de filtres de convolution à des cartes de caractéristiques redimensionnées présentant des paramètres de taille respectifs différents.

4. Système de DAO selon l'une quelconque des revendications précédentes, dans lequel le CNN est configuré pour effectuer une opération d'adaptation de dispositif en fonction d'un identifiant de dispositif d'un dispositif par lequel l'image 3D a été capturée ; dans lequel l'opération d'adaptation de dispositif comprend l'obtention de paramètres d'au moins une transformation affine pour ledit dispositif, les paramètres affines des transformations affines étant optimisés par rétropropagation de gradients de perte ; et l'application de la au moins une transformation affine pour pondérer à nouveau des caractéristiques dans une couche intermédiaire du CNN.

5. Système de DAO selon l'une quelconque des revendications précédentes, comprenant en outre un composant d'entraînement qui est configuré pour :

obtenir un ensemble de données d'entraînement comprenant une pluralité d'images d'entraînement 3D, chaque image d'entraînement 3D étant associée à une étiquette de classe globale ;
pour chaque image d'entraînement 3D :

générer une pluralité d'images d'entrée 2D à partir de l'image d'entraînement 3D, chaque image d'entrée 2D se voyant attribuer l'étiquette de classe globale ;
transmettre les images d'entrée 2D au CNN ; et

appliquer la rétropropagation à une fonction de perte pour un classificateur du CNN pour ainsi entraîner le CNN ;
facultativement dans lequel l'ensemble de données d'entraînement comprend en outre des images d'entraînement 2D, et dans lequel les images d'entraînement 2D sont transmises au CNN pour générer des prédictions de classe pour les images d'entraînement 2D.

6. Procédé de diagnostic assisté par ordinateur (DAO) mis en oeuvre par ordinateur, comprenant :

la réception d'une image médicale 3D ;
la génération d'une prédiction d'une ou plusieurs classes pour des coupes 2D de l'image médicale 3D en utilisant un réseau neuronal convolutif (CNN) 2D ;
la détermination, pour une classe cible desdites une ou plusieurs classes, des tranches qui appartiennent à la classe cible ;

**caractérisé en ce que**, pour chaque tranche identifiée, le procédé comprend la détermination d'une contribution de chaque pixel de la tranche identifiée à la classification de la tranche identifiée comme appartenant à la classe cible par :

la définition d'une perte de classification pour la classe cible à une première valeur, et pour toutes les autres classes à une seconde valeur qui est différente de la première valeur ;

le calcul, à partir de la perte de classification, de gradients d'erreur ;

la rétropropagation des gradients d'erreur vers la couche intermédiaire du CNN 2D ; et

la détermination, à partir du tenseur de gradient à la couche intermédiaire prédéterminée, d'une matrice de contribution d'entrée représentant les contributions relatives de régions respectives de la tranche 2D à la probabilité de classe de la classe cible ; et

la génération d'une carte thermique, à partir de la matrice de contribution d'entrée, qui fournit une indication visuelle des contributions de pixels respectifs, et **en ce que** le CNN 2D comprend :

un premier composant de réseau neuronal convolutif (CNN) configuré pour extraire un ensemble de cartes de caractéristiques primaires à partir de tranches 2D de l'image 3D;

un extracteur de caractéristiques multiéchelle (114) configuré pour générer un ensemble de cartes de caractéristiques secondaires à partir des cartes de caractéristiques primaires, dans lequel l'extracteur de caractéristiques multiéchelle comprend :

une pluralité de redimensionneurs (213), les redimensionneurs respectifs (213) étant configurés pour générer des cartes de caractéristiques redimensionnées respectives, chaque redimensionneur (213) étant **caractérisé par** un paramètre de taille différent ; et

une pluralité de filtres de convolution configurés pour générer les cartes de caractéristiques secondaires à partir des cartes de caractéristiques redimensionnées ;

un composant de mise en commun (116) configuré pour générer un vecteur de caractéristiques à partir des cartes de caractéristiques secondaires ; et

un classificateur (118) configuré pour générer une ou plusieurs prédictions de classe pour l'image 3D sur la base du vecteur de caractéristiques ;

dans lequel le composant de mise en commun (116) comprend une couche de mise en commun de type top-k qui est configurée pour :

déterminer les k premières valeurs des cartes de caractéristiques secondaires pour chacun d'une pluralité de canaux convolutifs, où $k >= 2$ ; et

calculer une moyenne pondérée des k premières valeurs.

**7.** Procédé de DAO selon la revendication 6, comprenant en outre le fait d'amener un écran à restituer la carte thermique sous forme de superposition sur la tranche identifiée.

**8.** Système de DAO selon la revendication 6 ou 7, dans lequel le composant de classification (104) est configuré pour appliquer le même ensemble de filtres de convolution à des cartes de caractéristiques redimensionnées présentant des paramètres de taille respectifs différents.

**9.** Système de DAO selon l'une quelconque des revendications 6 à 8, dans lequel le CNN est configuré pour effectuer une opération d'adaptation de dispositif en fonction d'un identifiant de dispositif d'un dispositif par lequel l'image 3D a été capturée, dans lequel l'opération d'adaptation de dispositif comprend l'obtention de paramètres d'au moins une transformation affine pour ledit dispositif, les paramètres affines des transformations affines étant optimisés par rétropropagation de gradients de perte ; et l'application de la au moins une transformation affine pour pondérer à nouveau des caractéristiques dans une couche intermédiaire du CNN.

**10.** Procédé de DAO selon l'une quelconque des revendications 6 à 9, comprenant en outre l'entraînement du CNN par :

l'obtention d'un ensemble de données d'entraînement comprenant une pluralité d'images d'entraînement 3D, chaque image d'entraînement 3D étant associée à une étiquette de classe globale ;

pour chaque image d'entraînement 3D :

la génération d'une pluralité d'images d'entrée 2D à partir de l'image d'entraînement 3D, chaque image d'entrée 2D se voyant attribuer l'étiquette de classe globale ;
la transmission des images d'entrée 2D au CNN ; et

l'application de la rétropropagation à une fonction de perte pour un classificateur du CNN pour ainsi entraîner le CNN ;
facultativement dans lequel l'ensemble de données d'entraînement comprend en outre des images d'entraînement 2D, et dans lequel les images d'entraînement 2D sont transmises au CNN pour générer des prédictions de classe pour les images d'entraînement 2D.

11. Support de stockage non volatile lisible par ordinateur sur lequel sont stockées des instructions pour amener au moins un processeur (628) à effectuer un procédé selon l'une quelconque des revendications 6 à 10.

Figure 1

114

Resized primary feature
maps 203 ($N*C*H_1*W_1$)

Secondary
feature maps
204

Slice 1

Slice 1

Primary
feature maps
202
($N*C*H*W$)

213

Slice 2

Slice 2

Resizer 1

Slice 1

214

Slice N

Slice 2

Slice N

Resized primary feature
maps 203 ($N*C*H_2*W_2$)

116

118

Slice N

201

112

Resizer 2

213

Slice 1

Slice 2

205

214

Slice 2

Slice N

Slice N

111

100

## Figure 2

Figure 3

CNN feature extractor 421        Bottom part 411        Top part 412

bottom                                                        top

+

430                     431
Middle features    Reweighted middle features

Detected Class Probabilities

$f_{d1}$

3D Image from device d        Bottom part 411

Top part 412

423

Classifier

Normal
$P(normal)$

DME
$P(DME)$

Drusen
$P(Drusen)$

CNV
$P(CNV)$

...

201

$f_{dC}$

432: $f_{d1}, \cdots, f_{dC}$
Feature affine transformations (specific to device d)

# Figure 4

400

Figure 5

Figure 6

```
┌────────────────────────────────────────────────────┐
│ OBTAIN A TRAINING DATA SET COMPRISING A PLURALITY OF │── 702
│           2D AND/OR 3D TRAINING IMAGES               │
└────────────────────────────────────────────────────┘
                          │
                          ▼
┌────────────────────────────────────────────────────┐
│              GET NEXT TRAINING IMAGE                 │── 704
└────────────────────────────────────────────────────┘
                          │
                          ▼
┌────────────────────────────────────────────────────┐
│      IF 3D IMAGE, GENERATE 2D IMAGES FROM 3D IMAGE   │── 706
└────────────────────────────────────────────────────┘
                          │
                          ▼
┌────────────────────────────────────────────────────┐
│     SEND 2D IMAGE(S) TO PRE-TRAINED CNN TO GENERATE  │── 708
│               PRIMARY FEATURE MAPS                   │
└────────────────────────────────────────────────────┘
                          │
                          ▼
┌────────────────────────────────────────────────────┐
│               RESIZE PRIMARY FEATURE MAPS           │── 710
└────────────────────────────────────────────────────┘
                          │
                          ▼
┌────────────────────────────────────────────────────┐
│   APPLY CONVOLUTION KERNELS TO GENERATE SECONDARY    │── 712
│                  FEATURE MAPS                        │
└────────────────────────────────────────────────────┘
                          │
                          ▼
┌────────────────────────────────────────────────────┐
│     APPLY TOP-K POOLING TO GENERATE FEATURE VECTOR   │── 714
└────────────────────────────────────────────────────┘
                          │
                          ▼
┌────────────────────────────────────────────────────┐
│        CLASS PREDICTION FROM FEATURE VECTOR          │── 716
└────────────────────────────────────────────────────┘
                          │
                          ▼
┌────────────────────────────────────────────────────┐
│           DETERMINE CLASSIFICATION LOSS             │── 718
└────────────────────────────────────────────────────┘
                          │
                          ▼
            N        ◇ ALL TRAINING      720
           ◀─────────  IMAGES DONE?
                          │ Y
                          ▼
┌────────────────────────────────────────────────────┐
│      OPTIMISE PARAMETERS BY BACKPROPAGATION         │── 722
└────────────────────────────────────────────────────┘
                          │
                          ▼
                  ╱ LEARNED      ╱── 730
                 ╱  PARAMETERS  ╱
                                            ↖ 700
```

## Figure 7

DME-0.959

Figure 8

DME-0.964

Figure 9

Figure 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018082443 A

**Non-patent literature cited in the description**

- **VALENTE et al.** *Computer Methods and Programs in Biomedicine,* 2016, vol. 124, ISSN 0169-2607, 91-107
- **KURUVILLA et al.** *Computer Methods and Programs in Biomedicine,* January 2014, vol. 113 (1), 202-209
- **SELVARAJU RAMPRASAATH R et al.** *Grad-CAM: Visual Explanations from Deep Networks via Gradient-Based Localization*
- **KOOMSUBHA THANABHAT et al.** *A character-level convolutional neural network with dynamic input length for Thai text categorisation*
- **MAX JADERBERG et al.** *Spatial Transformer Networks*
- **K. SIMONYAN ; A. ZISSERMAN.** Very Deep Convolutional Networks for Large-Scale Image Recognition. *International Conference on Learning Representations,* 2015

- **K. HE et al.** Deep Residual Learning for Image Recognition. *2016 IEEE Conference on Computer Vision and Pattern Recognition (CVPR), Las Vegas, NV,* 2016, 770-778
- **G. HUANG et al.** Densely Connected Convolutional Networks. *2017 IEEE Conference on Computer Vision and Pattern Recognition (CVPR), Honolulu, HI,* 2017, 2261-2269
- Deep CORAL: Correlation Alignment for Deep Domain Adaptation. **SUN et al.** Computer Vision - ECCV 2016 Workshops, ECCV 2016, Lecture Notes in Computer Science. Springer, vol. 9915
- **LI et al.** Thoracic disease identification and localization with limited supervis''on. *CVPR,* June 2018
- **LI et al.** Scale-Aware Trident Networks for Object Detection. *arXiv: 1901.01892,* January 2019
- **LI et al.** Thoracic disease identification and localization with limited supervision. *CVPR,* June 2018